# EUROPEAN PATENT SPECIFICATION

(11) **EP 2 467 152 B1**
(45) Date of publication and mention of the grant of the patent: **17.05.2017**
(21) Application number: 10747123.7
(22) Date of filing: 16.08.2010
(51) Int. Cl.: A61K 38/17, A61K 39/395, A61K 39/00, A61K 45/06, C07K 16/28, A61P 3/10, A61P 3/00, A61P 9/00, A61P 29/00

(54) **USE OF NKG2D INHIBITORS FOR TREATING CARDIOVASCULAR AND METABOLIC DISEASES, SUCH AS TYPE 2 DIABETES**
VERWENDUNG VON NKG2D-HEMMERN ZUR BEHANDLUNG VON HERZ-KREISLAUF- UND STOFFWECHSELERKRANKUNGEN WIE ETWA TYP-2-DIABETES
UTILISATION D'INHIBITEURS NKG2D POUR LE TRAITEMENT DE MALADIES CARDIOVASCULAIRES ET MÉTABOLIQUES, COMME LE DIABÈTE DE TYPE 2

(30) Priority: 17.08.2009 US 234425 P; 12.02.2010 US 304113 P
(43) Date of publication of application: 27.06.2012
(73) Proprietor: The Penn State Research Foundation, University Park, PA 16802 (US); The Regents of the University of California, Oakland, CA 94607 (US)
(72) Inventor: XIONG, Na, State College PA 16803 (US); XIA, Mingcan, State College PA 16803 (US); RAULET, David, H., Berkeley CA 94707 (US); PETERSEN, Jacob, Sten, DK-2920 Charlottenlund (DK); BÖDVARSDÓTTIR, Thóra, Brynja, DK-2950 Vedbæk (DK)
(74) Representative: Grünecker Patent- und Rechtsanwälte PartG mbB
(86) International application number: PCT/US2010/045627
(87) International publication number: WO 2011/022334

(56) References cited:
- WO-A2-2005/097160
- WO-A2-2008/014035
- US-A1- 2005 158 307
- NASHED BAHER ET AL: "Antiatherogenic effects of dietary plant sterols are associated with inhibition of proinflammatory cytokine production in apo E-KO mice", JOURNAL OF NUTRITION, vol. 135, no. 10, October 2005 (2005-10), pages 2438-2444, ISSN: 0022-3166

## Description

### FIELD OF THE INVENTION

The present invention relates to methods of treating type 2 diabetes and/or conditions that may be regulated or normalised via inhibition of NKG2D, such as cardiovascular diseases.

### BACKGROUND OF THE INVENTION

A large and growing number of people suffer from diabetes mellitus. Diabetes mellitus, also commonly known as diabetes, is a metabolic disorder that results in elevation of the blood glucose level because of relative or absolute deficiency in the pancreatic hormone insulin. Insulin is secreted from the pancreas into the blood in response to the blood glucose level and a major function is to direct blood glucose into body stores, whereby the blood glucose level is controlled.

Diabetes is known in the Type 1 and Type 2 forms. Type 1 diabetes is characterized by a progressive loss of pancreatic beta cells due to an unfavourable balance between the destructive autoimmune processes targeting the beta cells on one side and the regenerative capacity of these cells on the other side. This imbalance eventually leads to total loss of beta cells and endogenous insulin secretion. Type 1 diabetes accounts for 5-10 % of diabetes. Type 2 diabetes is characterized by insulin resistance and impaired beta cell function, which includes impaired first phase insulin release, reduced beta cell pulse mass and insulin deficiency. Type 2 diabetes is the most common form of the disease, accounting for 90-95 % of diabetes.

People with diabetes are two to four times more likely to develop cardiovascular disease than people without diabetes. Cardiovascular disease is the major complication and leading cause of death in people with type 2 diabetes despite considerable improvements in therapy over the past decades. An increased risk of heart attack, stroke and amputation of the lower limbs are the major causes of premature death in people with diabetes. A higher prevalence of cardiovascular disease risk factors including obesity, hypertension, dyslipidemia and physical inactivity are usually present in the presence of type 2 diabetes.

Cardiovascular diseases are the most serious human health concern and the number one cause of death in the United States (U.S.). According to the American Heart Association, the cost for cardiovascular disease prevention and therapy will be an astonishing $448.5 billion for 2008 in the U.S. alone, resulting in a huge financial burden for the society. Atherosclerosis is a form of cardiovascular diseases in which plaques, composed of fatty substances and cellular components, build up in the inner lining of arterial vessels. The disease is initiated by abnormal metabolites deposited in susceptible regions of artery of patients with dysfunctional metabolic conditions, such as diabetes, and progresses with the involvement of multiple factors, including immune activation and inflammation.

Abnormal metabolic conditions, such as diabetes, cause various cellular stress responses that induce tissue inflammation and immune cell activation, which in turn exacerbates the metabolic dysfunction, creating a vicious cycle that sustains the downstream outcomes that underlie disorders such as cardiovascular disease progression. However, molecular events linking these processes are not well understood, hindering efforts to modulate the immune system for the treatment of the metabolic dysfunction and cardiovascular disease complications.

US 2005/158307 discloses that compounds (such as antibodies) that prevent the activation of NKD2D receptors may be useful for treating immune or inflammatory disorders (such as type I diabetes).

WO 2008/014035 discloses methods and compositions for treating and/or preventing autoimmune and/or inflammatory diseases by impairing the expansion and function of autoreactive T cells, NK cells and/or NKT cells through modulation of NKG2D.

WO 2005/037160 discloses the use of an agent that reduces ligand induced NKG2D activation of glycosides for treating or preventing inflammatory syndromes.

Nashed B. et al. (J. Nutr. 135 (2005), p. 2438-44) discloses that plant sterols can inhibit pro-inflammatory cytokine production in ApoE knock-out mice.

Accordingly, there is a need for effective methods for the prediction, prevention and treatment of metabolic dysfunction-associated diseases, such as diabetes as well as cardiovascular diseases and disorders.

### SUMMARY OF THE INVENTION

The present invention concerns the discovery of a family of immune stimulatory molecules that are upregulated in patients and animals with type 2 diabetes and/or metabolic dysfunctions and that might play an important role in activation of the immune cells for vascular and liver inflammation, metabolic dysfunction and cardiovascular disease development. In particular, elevated levels of stimulatory ligands for NKG2D, a potent immune activating receptor, were detected in type 2 diabetic human patients as well as in animal models for dysfunctional lipid metabolism and atherosclerosis.

Accordingly, the present invention provides novel, compositions for treating type 2 diabetes and conditions that may be regulated or normalized via inhibition of NKG2D, such as cardiovascular disease. The present disclosure also relates to novel methods for detecting and diagnosing a predisposition to type 2 diabetes or cardiovascular disease development or the presence of type 2 diabetes or cardiovascular disease.

In one embodiment, the present invention provides a treatment for type 2 diabetes comprising administering to a subject in need thereof an agent that inhibits NKG2D activation or signaling. In another embodiment, the present invention provides a treatment for type 2 diabetes comprising administering to a subject in need thereof an agent that blocks the NKG2D ligand binding interaction. In another embodiment, the present invention provides a treatment for a condition that may be regulated or normalized via inhibition of NKG2D comprising administering to a subject in need thereof an agent that inhibits NKG2D activation or signaling, wherein the condition is selected from the group consisting of type 2 diabetes, cardiovascular disease, inflammatory disease and metabolic dysfunction-associated disease. In another embodiment, the present invention provides a treatment for a condition that may be regulated or normalized via inhibition of NKG2D comprising administering to a subject in need thereof an agent that blocks the NKG2D ligand binding interaction, wherein the condition is selected from the group consisting of type 2 diabetes, cardiovascular disease, inflammatory disease and metabolic dysfunction-associated disease. The NKG2D ligand is MICA/B, ULBP/RAET1, Multl, Rae-1delta, Rae-1epsilon, or H60b.

In another embodiment, the present invention relates to the use of a an agent that inhibits NKG2D activation or signaling in a subject for treating type 2 diabetes. In another embodiment, the present invention relates to the use of an agent that blocks the NKG2D ligand binding interaction in a subject for treating type 2 diabetes. In another embodiment, the present invention relates to the use of an agent that inhibits NKG2D activation or signaling in a subject for treating a condition that may be regulated or normalized via inhibition of NKG2D, wherein the condition is selected from the group consisting of type 2 diabetes, cardiovascular disease, inflammatory disease and metabolic dysfunction-associated diseases. In another embodiment, the present invention relates to the use of an agent that blocks the NKG2D ligand binding interaction in a subject for treating a condition that may be regulated or normalized via inhibition of NKG2D, wherein the condition is selected from the group consisting of type 2 diabetes, cardiovascular disease, inflammatory disease and metabolic dysfunction-associated disease. The NKG2D ligand is MICA/B, ULBP/RAET1, Multl, Rae-1delta, Rae-1epsilon, or H60b.

In some embodiments, the condition that may be regulated or normalized via inhibition of NKG2D is type 2 diabetes. In some embodiments, the condition is cardiovascular disease.

In some embodiments, the subject is human.

In some embodiments, the agent is selected from the group consisting of soluble NKG2D, an antibody or antigen-binding fragment thereof that specifically binds NKG2D, an NKG2D ligand and an inhibitor of NKG2D ligand activity or expression. In some embodiments, the agent is an antibody or antigen-binding fragment thereof that specifically binds NKG2D. In some embodiments, the antibody or antigen-binding fragment thereof is human or humanized. In some embodiments, the antibody or antigen-binding fragment thereof binds human NKG2D (hNKG2D). In some embodiments, the antibody or antigen-binding fragment thereof reduces NKG2D-mediated activation or signaling of an NKG2D-expressing cell. In some embodiments, the antibody or antigen-binding fragment thereof competes with at least one NKG2D ligand in binding to NKG2D. In some embodiments, the NKG2D ligand is MICA/B.

In some embodiments, administration of the agent results in at least one of the following in the subject: reduces blood glucose levels, improves glucose tolerance, lowers insulin resistance, reduces body weight, lowers blood pressure, lowers inflammation or reduces metabolic dysfunctions.

In some embodiments, the agent is administered intravenously, intraperitoneally or subcutaneously.

In some embodiments, agent the present invention further comprises at least one additional agent selected from the group consisting of antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. In some embodiments, the additional agent is selected from the group consisting of (a) a blood glucose lowering agent selected from the group consisting of GLP-1 and GLP-1 derivatives and analogues, Exendin-4 and Exendin-4 derivatives and analogues, amylin and amylin derivatives and analogues, sulphonylureas, biguanides, meglitinides (such as nateglinide and repaglinide), glucosidase inhibitors, DPP-IV (dipeptidyl peptidase-IV) inhibitors, SGLT2 inhibitors, SGLT1 inhibitors or agonists, gastrin and gastrin analogues and derivatives, FGF-21 (fibroblast growth factor 21) and FGF-21 derivatives and analogues, proton pump inhibitors such as lansoprazole, omeprazole, dexlansoprazole, esomeprazole, pantoprazole, and rabeprazole, RXR agonists, Cholestyramine, colestipol, probucol, dextrothyroxine, PPAR agonists, and adiponectin and adiponectin derivatives and analogues; (b) a blood lipid lowering or lipid metabolism modifying agent selected from the group consisting of antihyperlipidemic agents, HMG CoA inhibitors (statins) such as lovastatin, pravastatin and simvastatin, and fibrates such as gemfibrozil and clofibrate; (c) an agent that lowers food intake or increases energy expenditure selected from the group consisting of NPY (neuropeptide Y) antagonists, PYY (polypeptide YY) agonists, PP (pancreatic polypeptide) agonists, Y2 receptor agonists, Y4 receptor agonists, mixed Y2/Y4 receptor agonists, MC4 (melanocortin 4) agonists, orexin antagonists, glucagon and glucagon derivatives and analogues, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, histamine H3 antagonists, and CART (cocaine amphetamine regulated transcript) agonists; and (d) a blood pressure lowering agent selected from the group consisting of β-blockers such as alprenolol, atenolol, timolol, pindolol, propranolol and metoprolol, ACE (angiotensin converting enzyme) inhibitors such as benazepril, captopril, enalapril, fosinopril, lisinopril, alatriopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin.

In one embodiment, the present invention provides a composition comprising a therapeutically effective amount of an agent that inhibits NKG2D activation or signaling and a pharmaceutically acceptable carrier. In another embodiment, the present invention provides a composition comprising a therapeutically effective amount of an agent that blocks the NKG2D ligand binding interaction and a pharmaceutically acceptable carrier.

In some embodiments, the agent is useful for treating type 2 diabetes. In some embodiments, the agent is useful for treating a condition that may be regulated or normalized via inhibition of NKG2D.

In some embodiments, the condition is selected from the group consisting of type 2 diabetes, cardiovascular disease, inflammatory disease and metabolic dysfunction-associated disease. In some embodiments, the condition is type 2 diabetes. In some embodiments, the condition is cardiovascular disease.

In some embodiments, the agent is selected from the group consisting of soluble NKG2D, an antibody or antigen-binding fragment thereof that specifically binds NKG2D, an NKG2D ligand and an inhibitor of NKG2D ligand activity or expression. In some embodiments, the agent is an antibody or antigen-binding fragment thereof that specifically binds NKG2D. In some embodiments, the antibody or antigen-binding fragment thereof is human or humanized. In some embodiments, the antibody or antigen-binding fragment thereof binds human NKG2D (hNKG2D).

In some embodiments, the agent is an inhibitor of NKG2D ligand activity or expression. In some embodiments, the inhibitor of NKG2D ligand activity or expression is a MICA/B inhibitor. In some embodiments, the MICA/B inhibitor is MICA/B-specific siRNA.

In some embodiments, the compositions of the present invention further comprise at least one additional agent selected from the group consisting of antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. In some embodiments, the additional agent is selected from the group consisting of (a) a blood glucose lowering agent selected from the group consisting of GLP-1 and GLP-1 derivatives and analogues, Exendin-4 and Exendin-4 derivatives and analogues, amylin and amylin derivatives and analogues, sulphonylureas, biguanides, meglitinides (such as nateglinide and repaglinide), glucosidase inhibitors, DPP-IV (dipeptidyl peptidase-IV) inhibitors, SGLT2 inhibitors, SGLT1 inhibitors or agonists, gastrin and gastrin analogues and derivatives, FGF-21 (fibroblast growth factor 21) and FGF-21 derivatives and analogues, proton pump inhibitors such as lansoprazole, omeprazole, dexlansoprazole, esomeprazole, pantoprazole, and rabeprazole, RXR agonists, Cholestyramine, colestipol, probucol, dextrothyroxine, PPAR agonists, and adiponectin and adiponectin derivatives and analogues; (b) a blood lipid lowering or lipid metabolism modifying agent selected from the group consisting of antihyperlipidemic agents, HMG CoA inhibitors (statins) such as lovastatin, pravastatin and simvastatin, and fibrates such as gemfibrozil and clofibrate; (c) an agent that lowers food intake or increases energy expenditure selected from the group consisting of NPY (neuropeptide Y) antagonists, PYY (polypeptide YY) agonists, PP (pancreatic polypeptide) agonists, Y2 receptor agonists, Y4 receptor agonists, mixed Y2/Y4 receptor agonists, MC4 (melanocortin 4) agonists, orexin antagonists, glucagon and glucagon derivatives and analogues, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, histamine H3 antagonists, and CART (cocaine amphetamine regulated transcript) agonists; and (d) a blood pressure lowering agent selected from the group consisting of β-blockers such as alprenolol, atenolol, timolol, pindolol, propranolol and metoprolol, ACE (angiotensin converting enzyme) inhibitors such as benazepril, captopril, enalapril, fosinopril, lisinopril, alatriopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin.

Also disclosed is a kit comprising: (a) a therapeutically effective amount of an agent that inhibits NKG2D activation or signaling combined with a pharmaceutically acceptable carrier; and (b) instructions for use. In another embodiment, the present invention provides a kit comprising: (a) a therapeutically effective amount of an agent that blocks the NKG2D ligand binding interaction combined with a pharmaceutically acceptable carrier; and (b) instructions for use.

The agent is useful for treating type 2 diabetes.

The agent is useful for treating a condition that may be regulated or normalized via inhibition of NKG2D.

The condition is selected from the group consisting of type 2 diabetes, cardiovascular disease, inflammatory disease and metabolic-dysfunction associated disease. Preferably , the condition is type 2 diabetes or the condition is cardiovascular disease.

The agent is selected from the group consisting of soluble NKG2D, an antibody or antigen-binding fragment thereof that specifically binds NKG2D, an NKG2D ligand and an inhibitor of NKG2D ligand activity or expression. Preferably , the agent is an antibody or antigen-binding fragment thereof that specifically binds NKG2D. Preferably, the antibody or antigen-binding fragment thereof is human or humanized. Preferably, the antibody or antigen-binding fragment thereof binds human NKG2D (hNKG2D).

The kits may further comprise at least one additional agent selected from the group consisting of antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. Preferably, additional agent is selected from the group consisting of (a) a blood glucose lowering agent selected from the group consisting of GLP-1 and GLP-1 derivatives and analogues, Exendin-4 and Exendin-4 derivatives and analogues, amylin and amylin derivatives and analogues, sulphonylureas, biguanides, meglitinides (such as nateglinide and repaglinide), glucosidase inhibitors, DPP-IV (dipeptidyl peptidase-IV) inhibitors, SGLT2 inhibitors, SGLT1 inhibitors or agonists, gastrin and gastrin analogues and derivatives, FGF-21 (fibroblast growth factor 21) and FGF-21 derivatives and analogues, proton pump inhibitors such as lansoprazole, omeprazole, dexlansoprazole, esomeprazole, pantoprazole, and rabeprazole, RXR agonists, Cholestyramine, colestipol, probucol, dextrothyroxine, PPAR agonists, and adiponectin and adiponectin derivatives and analogues; (b) a blood lipid lowering or lipid metabolism modifying agent selected from the group consisting of antihyperlipidemic agents, HMG CoA inhibitors (statins) such as lovastatin, pravastatin and simvastatin, and fibrates such as gemfibrozil and clofibrate; (c) an agent that lowers food intake or increases energy expenditure selected from the group consisting of NPY (neuropeptide Y) antagonists, PYY (polypeptide YY) agonists, PP (pancreatic polypeptide) agonists, Y2 receptor agonists, Y4 receptor agonists, mixed Y2/Y4 receptor agonists, MC4 (melanocortin 4) agonists, orexin antagonists, glucagon and glucagon derivatives and analogues, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, histamine H3 antagonists, and CART (cocaine amphetamine regulated transcript) agonists; and (d) a blood pressure lowering agent selected from the group consisting of β-blockers such as alprenolol, atenolol, timolol, pindolol, propranolol and metoprolol, ACE (angiotensin converting enzyme) inhibitors such as benazepril, captopril, enalapril, fosinopril, lisinopril, alatriopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin..

Also disclosed is a method of detecting a predisposition to developing type 2 diabetes or the presence of type 2 diabetes in a subject comprising: (a) obtaining a sample from the subject; (b) contacting the sample with at least one reagent that detects the presence of MICA/B expression; (c) measuring the level of MICA/B expression in the sample; and (d) correlating overexpression of MICA/B with a predisposition to type 2 diabetes development or the presence of type 2 diabetes in the subject. Also disclosed is a method of detecting a predisposition to developing cardiovascular disease or the presence of cardiovascular disease in a subject comprising: (a) obtaining a sample from the subject; (b) contacting the sample with at least one reagent that detects the presence of MICA/B expression; (c) measuring the level of MICA/B expression in the sample; and (d) correlating overexpression of MICA/B with a predisposition to cardiovascular disease development or the presence of cardiovascular disease in the subject.

Preferably, the reagent is a MICA/B antibody.

Preferably, the sample is serum.

Preferably, the subject is human.

Preferably, the methods further comprise measuring the level of expression of a cardiovascular disease marker that is not MICA/B in the sample and correlating overexpression or underexpression of the cardiovascular disease marker with a predisposition to cardiovascular disease development or presence of cardiovascular disease in the subject.

### BRIEF DESCRIPTION OF THE DRAWINGS

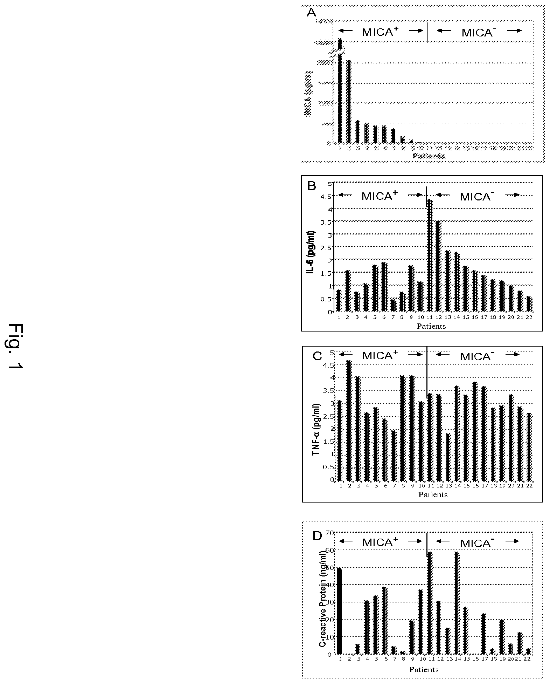

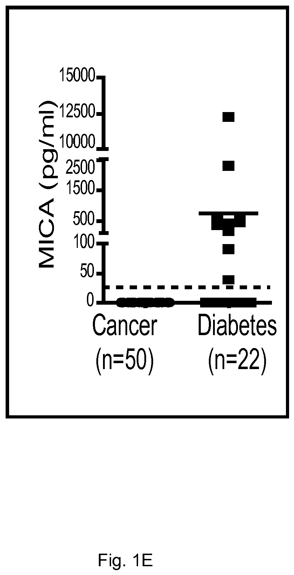

**FIG. 1** is a series of graphs showing detection of soluble MICA proteins in sera of type 2 diabetic patients and its association with expression of other pro-inflammatory cytokines. **(A)** Detection of soluble MICA in the blood of type 2 diabetic patients. Sera of the diabetic patients were assayed for MICA proteins using an ELISA kit (R&D Systems, Minneapolis, MN). Recombinant MICA proteins were used as standards, based on which the serum MICA levels were calculated. On the x-axis, each number represents a patient. **(B and C)** IL-6 and TNF-α levels in the sera of the same patients, analyzed by a human pro-inflammatory 4-plex panel (IL-6, TNF-α, IL-1β and IFN-γ) (Meso Scale Discovery, Gaithersburg, MD). **(D)** Sera levels of C-reactive protein (CRP) in the sera of the type 2 diabetic patients as analyzed by a CRP ELISA kit (Calbiotech Inc. Spring Valley, CA). **(E)** Diabetes patients on average have higher MICA levels in their sera than fifty cancer patients that were tested. Data for the type 2 diabetic patients is grouped and compared with the data from the fifty cancer patients.

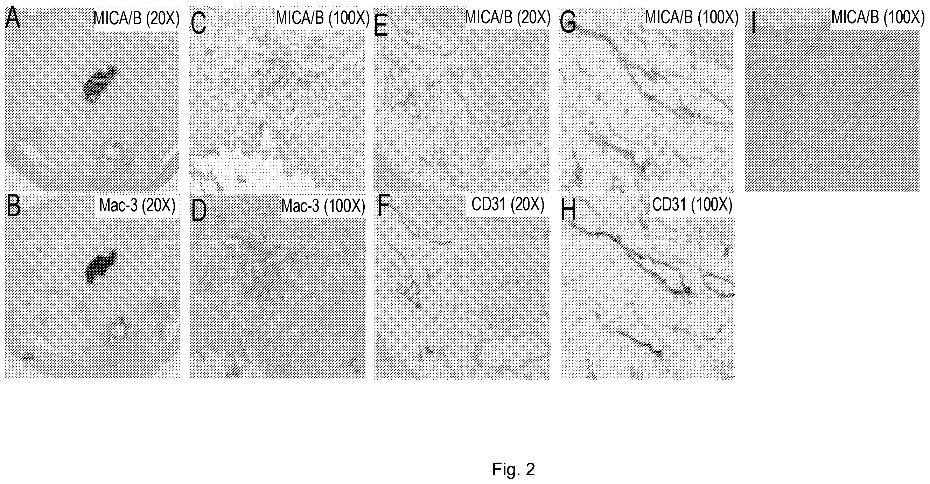

**FIG. 2** is a series of micrographs of cryosections showing detection of MICA/B by immunohistochemical staining on human aortic plaques. Cryosections of the plaques were stained with anti-MICA/B antibodies **(A, C, E, G).** Adjacent sections were stained with anti-Mac-3 or CD31 antibodies to reveal macrophages or endothelial cells **(B, D, F, H).** A section of normal aorta was used as a control for MICA staining **(I).**

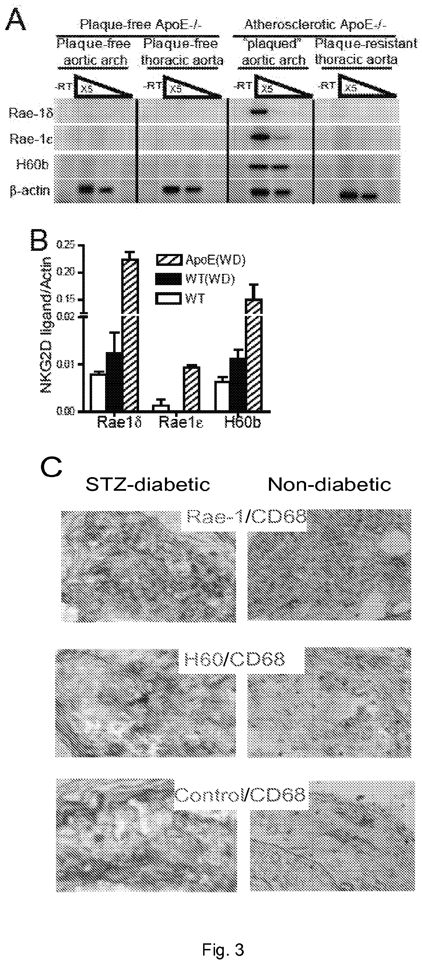

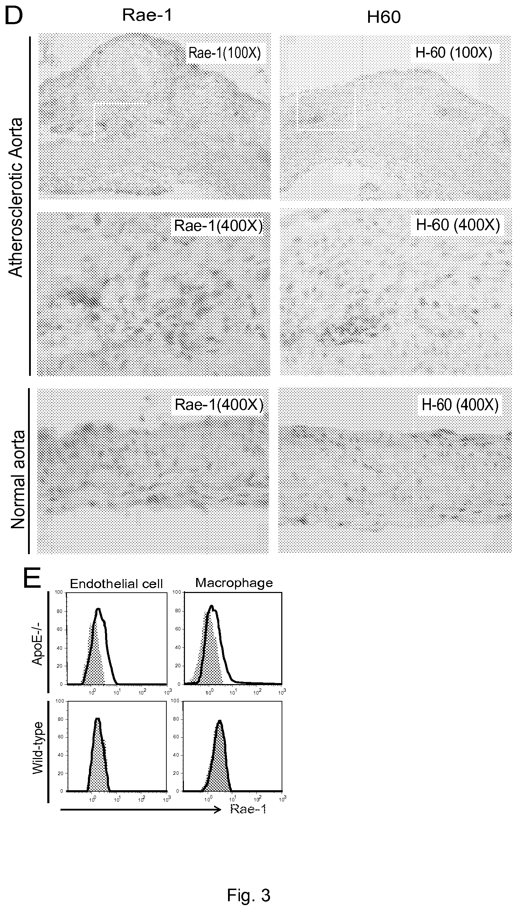

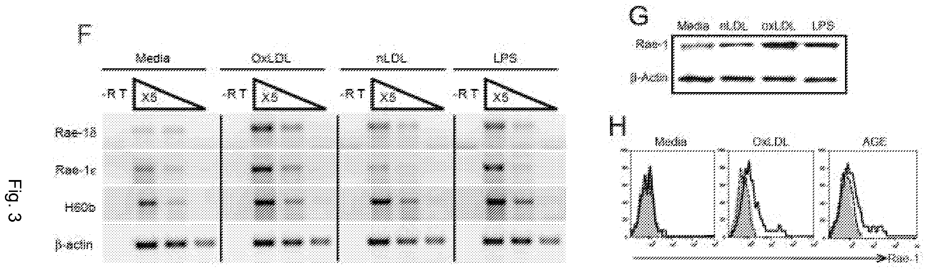

**FIG. 3** is a series of photographs of electrophoretic gels and a series of photographs of immunohistochemical-stained cryosections demonstrating upregulation of NKG2D ligands in atherosclerotic plaques of ApoE-/- mice. **(A)** Semi-quantitative RT-PCR analysis of transcripts for NKG2D ligands Rae-1δ, Rae-1ε and H60b in RNAs of aortic plaque lesions. **(B)** Real-time RT-PCR analysis of Rae-1δ, Rae-1ε and H60b transcripts in RNA samples isolated from aortic arch regions of Western diet (WD)-fed ApoE-/- and age-matched Western diet and normal chow-fed wild type mice. The experiment was done twice. **(C)** Immunohistochemical staining of cryosections of plaques from ApoE-/- mice that developed the plaques naturally (right) or accelerated by STZ-induced diabetes (left). The sections were co-stained with anti-Rae-1 or H60 antibody (brown) and anti-CD68 antibody (blue). **(D)** Immunohistochemical staining of atherosclerotic aortae of Western diet-fed ApoE-/- mice for NKG2D ligands Rae-1 and H60. Cryosections of atherosclerotic aortic arch regions (top and middle) and "plaque-free" thoracic regions (bottom) of the same Western diet-fed *ApoE-*/*-* mice were stained with polyclonal goat anti-mouse anti-Rae-1 (R&D Systems) or H60 antibodies (Santa Cruz Biotechnology). The pictures in the middle were higher amplifications (400X) of the boxed areas of the top panels (100X). **(E)** Flow cytometric analysis of cell surface Rae-1 expression on macrophages and endothelial cells isolated from atherosclerotic aortae of *Apo-E*^{*-*/*-*}mice or normal aortae of wild type mice. The macrophages were gated on the CD45⁺CD11b⁺CD16/32⁺ population and endothelial cells were gated on the CD45-CD31⁺ population. The gray areas indicated isotype-matched control antibody staining. **(F)** Western blot analysis for total Rae-1 proteins in the in vitro treated wild-type macrophages. **(G)** Semi-quantitative RT-PCR analysis for the different NKG2D ligand upregulation in wild-type macrophages cultured in medium only and in presence of additional oxLDL, natural LDL (nLDL) and LPS (as a control). **(H)** Flow cytometric analysis of Rae-1 expression on wild-type macrophages in vitro cultured in presence of oxidized LDL (oxLDL) or advanced glycation end products (AGE). Macrophages were isolated from peritoneal cavity and cultured in vitro for two days in media containing 5 µg/ml oxLDL or 200 µg/ml AGE before analyzed by flow cytometry similarly as in the panel E except that the dashed lines were of staining in the presence of 10-fold excess of unlabeled anti-pan-Rae-1 antibodies.

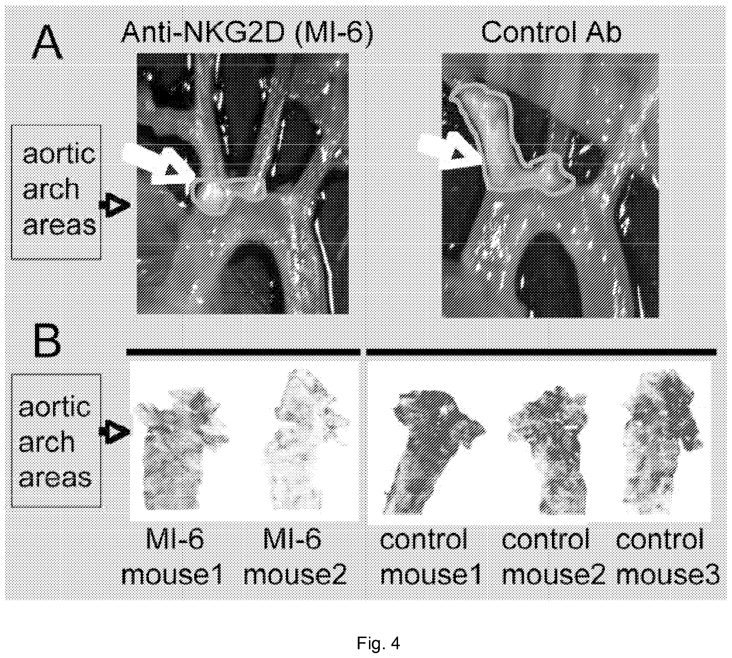

**FIG. 4** is a series of photographs of aorta from ApoE-null mice showing suppression of plaque formation by anti-NKG2D antibody in diabetic ApoE-null mice. **(A)** Plaque formation in aorta of representative anti-NKG2D or control antibody treated diabetic ApoE-null mice. Aortae were perfused with PBS and observed under a dissection microscope for apparent opaque plaques. Note the dramatically decreased sizes of the plaques in the anti-NKG2D antibody-treated mouse (circled areas). **(B)** En face Oil red O staining of aorta for deposition of lipid contents in anti-NKG2D (MI-6) and control antibody (control) treated ApoE-/- mice from one representative experiment. Aortae were opened up, fixed in 10% formalin and stained with Oil red O dye. Lipid contents stained dark red. Note significantly less Oil red O staining in aortic arch areas of the anti-NKG2D antibody treated mice.

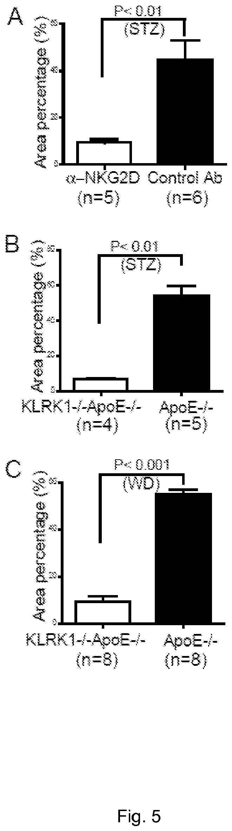

**FIG. 5** is a series of graphs demonstrating that targeting the NKG2D/ligand interaction prevents atherosclerosis in various mouse models. **(A)** The effect of anti-NKG2D antibody blockage on the atherosclerosis development in streptozotocin-induced diabetic ApoE-/- mice. Sizes of plaques on aorta of control and anti-NKG2D treated diabetic ApoE-/- mice were calculated based on ratios of the Oil red O positive staining areas of total arch areas. **(B and C)** The effect of NKG2D knockout on atherosclerosis development in streptozotocin-induced diabetic **(B)** or Western diet-fed **(C)** KLRK1-/-ApoE-/- mice (the KLRK1 gene encodes the NKG2D protein). The plaque sizes were calculated the same way as the panel A. ApoE-/- mice were used as controls.

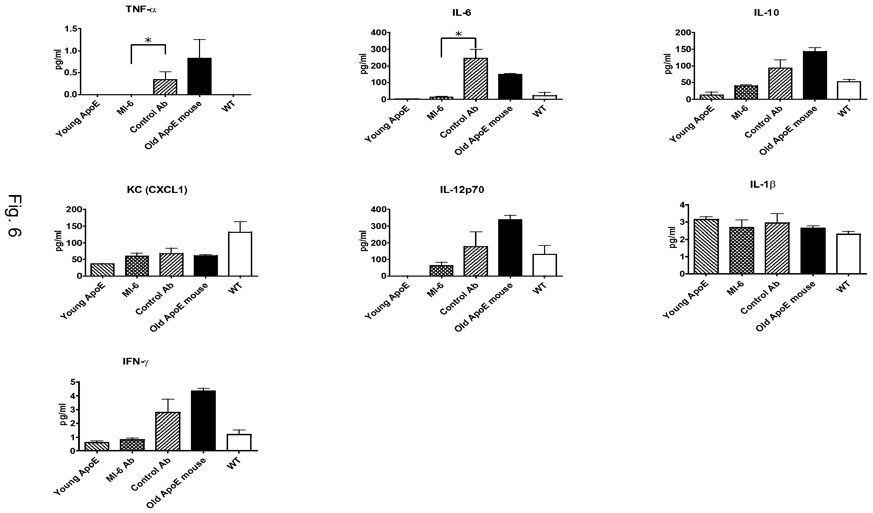

**FIG. 6** is a series of graphs showing reduced pro-inflammatory cytokines in the sera of anti-NKG2D antibody treated diabetic ApoE null mice. Sera of four anti-NKG2D (MI-6 Ab)-treated and three control antibody (Con Ab)-treated diabetic ApoE null mice, along with two each of age-matched wild type B6 (Wild Type), one month (Young ApoE) or eight month old (Old ApoE) ApoE-/- mice were analyzed directly on a mouse proInflammatory-7plex kit (IL-6, TNF-α, IFN-γ, IL-12p70, IL-10, IL-1β and KC/CXCL1) (Meso Scale Discovery, Gaithersburg, MD). The star * indicate statistically significant differences in the values between the anti-NKG2D antibody and control antibody treated diabetic ApoE-/- mice.

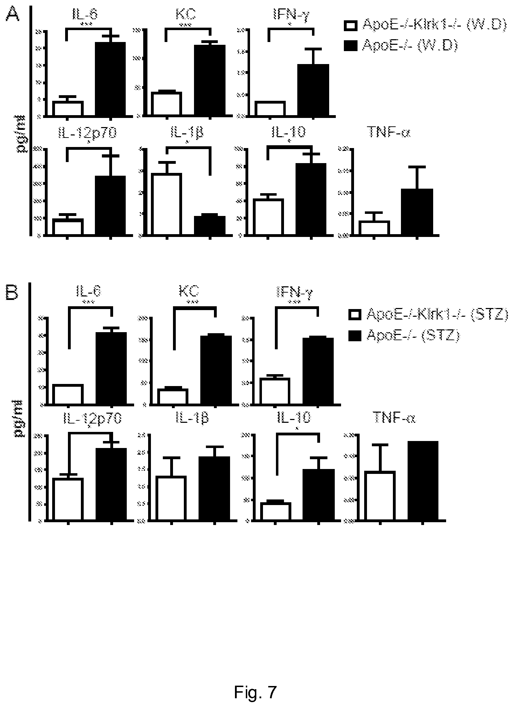

**FIG. 7** is a series of graphs showing reduced pro-inflammatory cytokines in the sera of KLRK1-/-ApoE-/- mice (which have a defective NKG2D gene). Sera of western diet fed (12 mice/group) (**panel A**) or STZ-induced diabetic (4 mice/group) (**panel B**) KLRK1-/-ApoE-/- and ApoE-/- mice were analyzed directly by a mouse prolnflammatory-7plex kit (IL-6, TNF-α, IFN-γ, IL-12p70, IL-10, IL-1β and KC/CXCL1) (Meso Scale Discovery, Gaithersburg, MD) as in Figure 6. The stars indicate statistically significant differences in the values between the anti-NKG2D antibody and control antibody treated diabetic ApoE-/- mice (***p<0.01; *p<0.05).

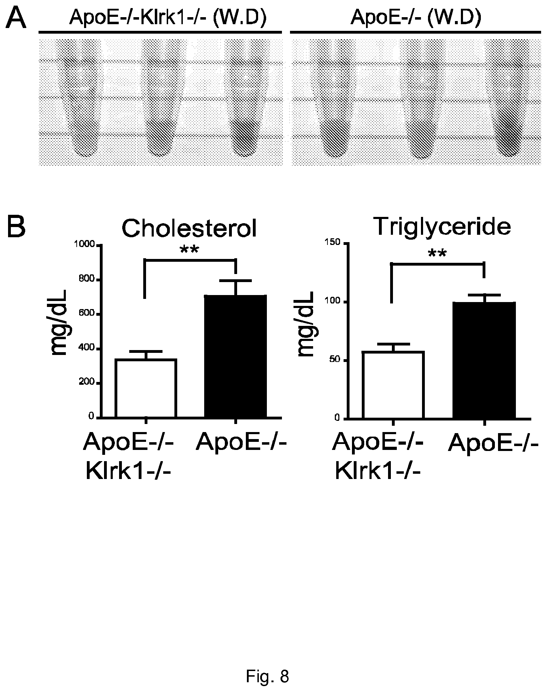

**FIG. 8** displays a pair of photographs of sera and a pair of graphs showing alleviated metabolic conditions in KLRK1-/-ApoE-/- mice. **(A)** Clearer appearance of sera of western diet fed KLRK1-/-ApoE-/- than the similarly fed ApoE-/- mice. **(B)** Reduced cholesterol and triglyceride levels in blood of KLRK1-/-ApoE-/- mice. Sera of the panel A were analyzed for total cholesterol and triglyceride. The stars ** indicate statistically significant differences in the values between the KLRK1/ApoE double knockout and ApoE-knockout mice (**p<0.05). For each group, at least 5 mice were used.

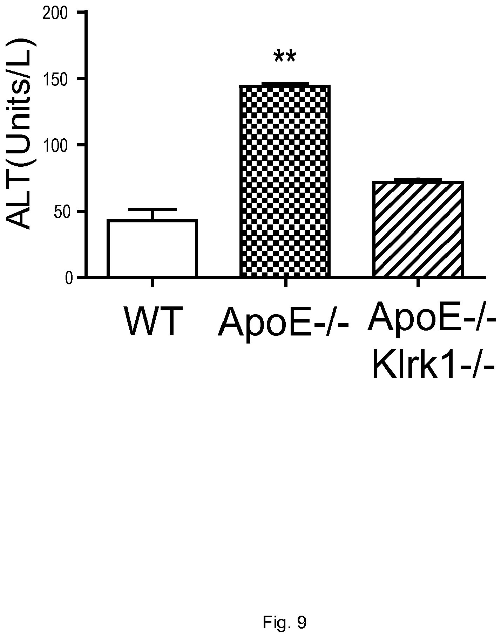

**FIG. 9** is a graph showing reduced serum alanine aminotransferase (ALT) activities in NKG2D-knockout KLRK1-/-ApoE-/- mice compared to ApoE-/- mice. For each group, at least 5 mice were used.

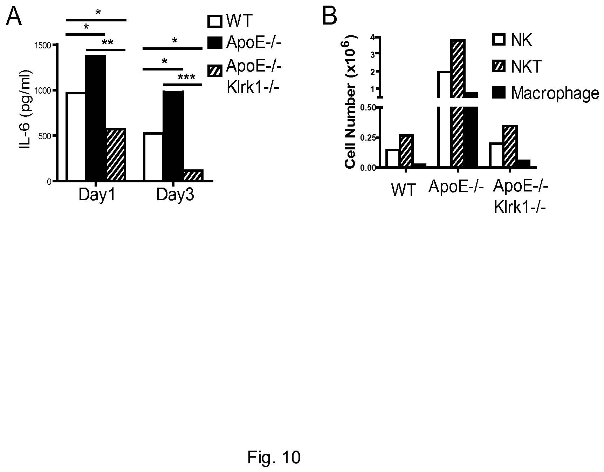

**FIG. 10** is a pair of graphs showing that preventing the NKG2D/ligand interaction reduced the inflammation of livers of Western diet fed ApoE-/- mice. **(A)** Reduced IL-6 production by ex vivo cultured liver explants of NKG2D-knockout KLRK1-/-ApoE-/- mice. PBS-perfused livers were excised from Western diet fed ApoE-/- or NKG2D-knockout KLRK1-/-ApoE-/- mice. Equal weights of excised livers were cut into about 1mm³ cubes and cultured in media for 1 or 3 days. The culture media were recovered and analyzed for cytokines by ELISA. The experiment was repeated twice. **(B)** Reduced immune cell infiltration in livers of Western diet fed NKG2D-knockout KLRK1-/-ApoE-/- mice. The numbers are of each type of immune cells per liver, calculated based on total mononucleocytes isolated from livers and percentages of each type. For each group, at least 3 mice were used. One representative of three independent experiments was shown.

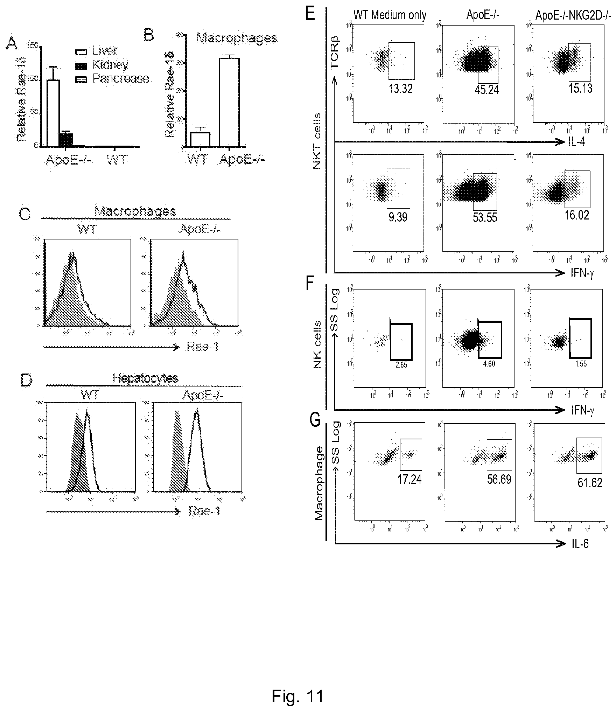

**FIG. 11** is a series of graphs and fluorescence activated cell sorting analysis plots showing that NKG2D ligands were upregulated in the cells of livers of Western diet fed ApoE-/- mice and that preventing the NKG2D/ligand interaction reduced the activation of NKG2D-expressing immune cells in the livers. **(A)** Real-time RT-PCR analysis of Rae-1δ transcripts in livers and other organs of 12-week-old male ApoE-/- mice fed on a Western diet for 8 weeks and wild type mice of same ages. **(B)** Real-time RT-PCR analysis of Rae-1δ transcripts in purified macrophages of livers of Western diet fed ApoE-/- mice and wild type mice. **(C and D)** Flow cytometric analysis of Rae-1 expression on liver macrophages **(C)** and hepatocytes **(D)** of Western diet fed ApoE-/- mice. **(E)** Reduced production of IFN-γ and IL-4 by the liver NKT cells of NKG2D-knockout KLRK1-/-ApoE-/- mice. **(F)** Lower IFN-γ production by liver NK cells of NKG2D-knockout KLRK1-/-ApoE-/- than of ApoE-/- mice. **(G)** No difference in IL-6 production by liver macrophages of NKG2D-knockout KLRK1-/-ApoE-/- and ApoE-/- mice.

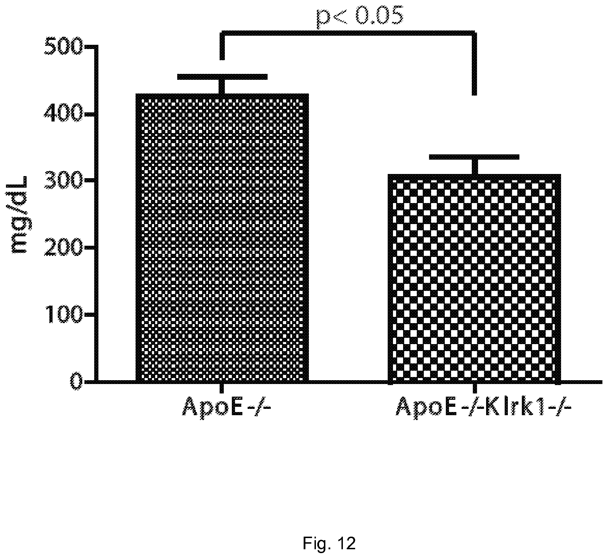

**FIG. 12** is a graph demonstrating that targeting NKG2D suppresses type 2 diabetes. The 8-week old ApoE-/- and NKG2D-deficient ApoE-/-KLRK1-/- mice were fed the Western diet for 3 months. Blood sera were then collected and assessed for glucose levels. The glucose levels of the ApoE-/- and ApoE-/-KLRK1-/- mice were the averages of six mice in each group. The difference between the two groups is statistically significant (p< 0.05).

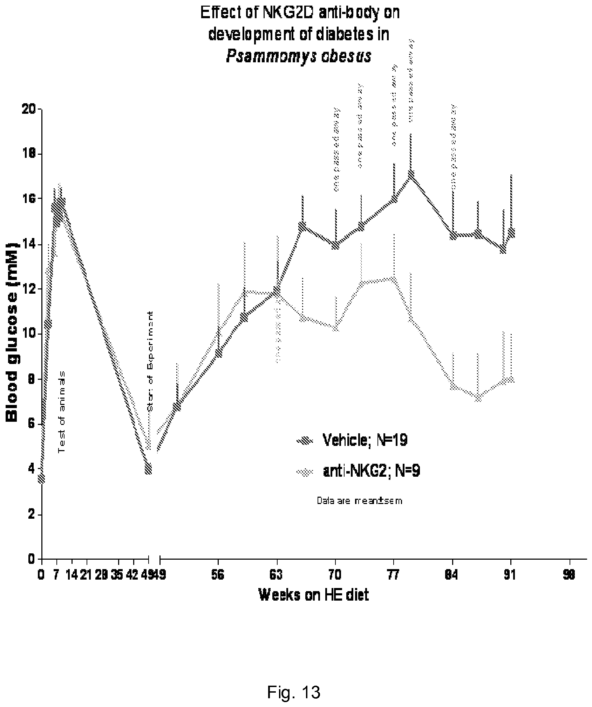

**FIG. 13** shows the effect of anti-NKG2D antibody on the development of diabetes in the gerbil *Psammomys obesus* (also known as sand rats), an animal model of type 2 diabetes. Male and female *P. obesus* (Harlan, Jerusalem, Israel) were fed low energy (2.4 kcal/g) chow until the age of 9 weeks where they were transferred to an ad libitum high energy (3.1kcal/g) diet during which body weight (BW) and morning blood glucose (mBG) was followed for up to 10 days (induction period). The animals that showed increased mBG levels, defined as mBG >10 mmol/L on two consecutive readings, were transferred back to low energy diet and were used in the actual study (prevention mode) 10 days later when their mBG levels were back to non-diabetic levels. The animals that did not show any increase in mBG during the induction period were sacrificed and not used further. (Data not shown.) *P. obesus* were treated with vehicle (N=19) or NKG2D-PE antibody (clone CX5) (N=9), once weekly by intra-peritoneal injection. The study was conducted over 6 weeks during which mBG and BW were followed regularly. All of the animals in the vehicle group became severely diabetic with a mean mBG of 14.5 ± 2.6 mM (mean ± sem) while the animals treated with the NKG2D antibody remained normoglycaemic (mBG<8 mM) and had a significantly lower mBG of 8.0 ± 1.9 mM, p<0.0001 (mean ± sem). Treatment with NKG2D antibody totally inhibited the development of type 2 diabetes in *P.obesus* and five animals in the vehicle group had to be sacrificed during the study due to severe diabetic ketoacidosis. There was no difference in BW gain in the two treatment groups.

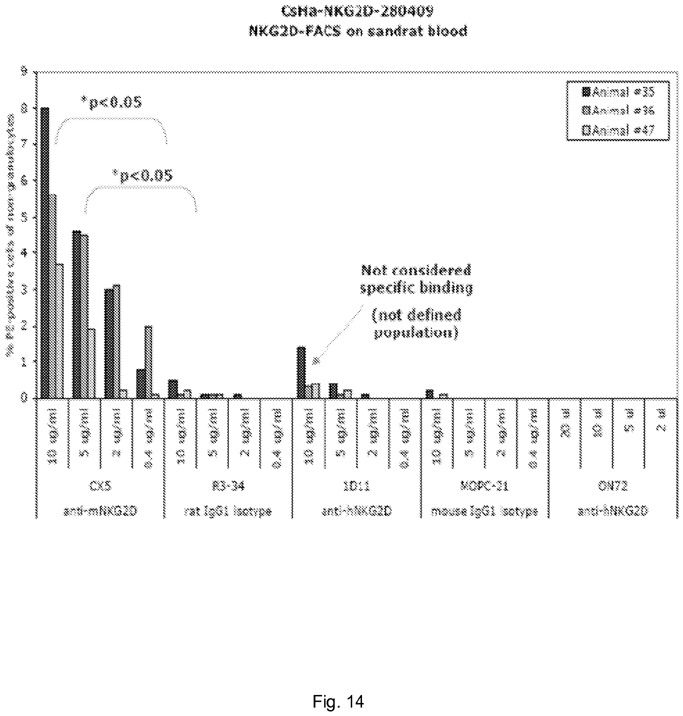

**FIG. 14** shows the flow cytometric analysis of NKG2D antibody binding to blood cells from *Psammomys obesus* (sand rats), an animal model of type 2 diabetes. A dose-dependent binding was observed for the anti-mouse NKG2D-PE antibody (clone CX5) to NK cells in sand rat blood. This binding was specific as there was no binding of the isotype control antibodies (rat IgG1-PE isotype control (R3-34) and mouse IgG1-PE isotype control (MOPC-21)) or the anti-human NKG2D antibodies (clone 1 D11 and clone ON72).

### DEFINITIONS

Unless otherwise defined, all technical terms used herein have the same meaning as commonly understood by one of ordinary skill in the art to which this invention belongs.

As used herein, "protein" and "polypeptide" are used synonymously to mean any peptide-linked chain of amino acids, regardless of length or post-translational modification, e.g., glycosylation or phosphorylation.

As used herein, the terms "MICA/B proteins" or "MICA/B polypeptide" is meant an expression product of an MHC class I chain-related A or B gene such as the native human MICA protein (accession no. L14848), or a protein that shares at least 65% (but preferably 75, 80, 85, 90, 95, 96, 97, 98, or 99%) amino acid sequence identity with the foregoing and displays a functional activity of a native MICA protein, or the native human MICB protein (accession no. NM_005931), or a protein that shares at least 65% (but preferably 75, 80, 85, 90, 95, 96, 97, 98, or 99%) amino acid sequence identity with the foregoing and displays a functional activity of a native MICB protein. A "functional activity" of a protein is any activity associated with the physiological function of the protein. For example, functional activities of a native MICA/B protein may include binding to NKG2D and inducing immune activation, such as secretion of cytokines.

As used herein, the terms "NKG2D protein" or "NKG2D polypeptide" is meant an expression product of a KLRK1 gene such as the native human NKG2D protein (accession no. 574240 or a protein that shares at least 65% (but preferably 75, 80, 85, 90, 95, 96, 97, 98, or 99%) amino acid sequence identity with the foregoing and displays a functional activity of a native NKG2D protein. For example, functional activities of a native NKG2D protein may include binding to its ligands and transducing signals into immune cells to modulate their gene expression and activation.

As used herein, the term "gene" is meant a nucleic acid molecule that codes for a particular protein, or in certain cases, a functional or structural RNA molecule.

As used herein, the terms "MICA/B gene," "MICA gene," "MICB gene," "MICA/B polynucleotide," "MICA polynucleotide," "MICB polynucleotide," "MICA/B nucleic acid," "MICA nucleic acid," or "MICB nucleic acid" is meant a native human MICA or MICB-encoding nucleic acid sequence, e.g., the native human MICA gene (accession no. L14848); e.g., the native human MICB gene (accession no. NM_005931), a nucleic acid having sequences from which a MICA or MICB cDNA can be transcribed; and/or allelic variants and homologs of the foregoing. The terms encompass double-stranded DNA, single-stranded DNA, and RNA.

As used herein, "KLRK1," "Klrk1," "KLRK1 gene," "Klrk1 gene," " NKG2D gene," "NKG2D polynucleotide," or "NKG2D nucleic acid" is meant a native human NKG2D-encoding nucleic acid sequence, e.g., the native human KLRK1 gene (accession no. 574240); a nucleic acid having sequences from which a KLRK1 cDNA can be transcribed; and/or allelic variants and homologs of the foregoing. The terms encompass double-stranded DNA, single-stranded DNA, and RNA.

As used herein, a "nucleic acid" or a "nucleic acid molecule" means a chain of two or more nucleotides such as RNA (ribonucleic acid) and DNA (deoxyribonucleic acid). Nucleic acid molecules as described herein may be in the form of RNA or in the form of DNA (e.g., cDNA, genomic DNA, and synthetic DNA). The DNA may be double-stranded or single-stranded, and if single-stranded may be the coding (sense) strand or non-coding (anti-sense) strand.

As used herein, the terms "patient," "subject" and "individual" are used interchangeably herein, and mean a mammalian (e.g., human) subject to be treated and/or to obtain a biological sample from.

As used herein, "bind," "binds," or "interacts with" means that one molecule recognizes and adheres to a particular second molecule in a sample or organism, but does not substantially recognize or adhere to other structurally unrelated molecules in the sample.

As used herein, the term "labeled," with regard to a probe or antibody, is intended to encompass direct labeling of the probe or antibody by coupling (i.e., physically linking) a detectable substance to the probe or antibody.

As used herein, when referring to a nucleic acid molecule or polypeptide, the term "native" refers to a naturally-occurring (e.g., a WT) nucleic acid or polypeptide.

As used herein, the terms "diagnostic," "diagnose" and "diagnosed" mean identifying the presence or nature of a pathologic condition.

As used herein, the term "sample" is used herein in its broadest sense. A sample including polynucleotides, peptides, antibodies and the like may include a bodily fluid, a soluble fraction of a cell preparation or media in which cells were grown, genomic DNA, RNA or cDNA, a cell, a tissue, skin, hair and the like. Examples of samples include saliva, serum, biopsy specimens, blood, urine, and plasma.

As used herein, "sequence identity" means the percentage of identical subunits at corresponding positions in two sequences when the two sequences are aligned to maximize subunit matching, i.e., taking into account gaps and insertions. Sequence identity is present when a subunit position in both of the two sequences is occupied by the same nucleotide or amino acid, e.g., if a given position is occupied by an adenine in each of two DNA molecules, then the molecules are identical at that position. For example, if 7 positions in a sequence 10 nucleotides in length are identical to the corresponding positions in a second 10-nucleotide sequence, then the two sequences have 70% sequence identity. Sequence identity is typically measured using sequence analysis software (e.g., Sequence Analysis Software Package of the Genetics Computer Group, University of Wisconsin Biotechnology Center, 1710 University Avenue, Madison, Wis. 53705).

When referring to mutations in a nucleic acid molecule, "silent" changes are those that substitute one or more base pairs in the nucleotide sequence, but do not change the amino acid sequence of the polypeptide encoded by the sequence. "Conservative" changes are those in which at least one codon in the protein-coding region of the nucleic acid has been changed such that at least one amino acid of the polypeptide encoded by the nucleic acid sequence is substituted with another amino acid having similar characteristics.

As used herein, the terms "oligonucleotide", "siRNA" "siRNA oligonucleotide" and "siRNA's" are used interchangeably throughout the specification and include linear or circular oligomers of natural and/or modified monomers or linkages, including deoxyribonucleosides, ribonucleosides, substituted and alpha-anomeric forms thereof, peptide nucleic acids (PNA), locked nucleic acids (LNA), phosphorothioate, methylphosphonate, and the like. Oligonucleotides are capable of specifically binding to a target polynucleotide by way of a regular pattern of monomer-to-monomer interactions, such as Watson-Crick type of base pairing, Hoögsteen or reverse Hoögsteen types of base pairing, or the like.

As used herein, the term "antibody" is used in the broadest sense and specifically includes full-length monoclonal antibodies, polyclonal antibodies, and, unless otherwise stated or contradicted by context, antigen-binding fragments, antibody variants, and multispecific molecules thereof, so long as they exhibit the desired biological activity. Generally, a full-length antibody is a glycoprotein comprising at least two heavy (H) chains and two light (L) chains inter-connected by disulfide bonds, or an antigen binding portion thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarily determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. General principles of antibody molecule structure and various techniques relevant to the production of antibodies are provided in, *e.g*., Harlow and Lane, ANTIBODIES: A LABORATORY MANUAL, Cold Spring Harbor Laboratory Press, Cold Spring Harbor, N.Y., (1988). For example, anti-NKG2D antibodies as described herein are capable of binding portions of NKG2D that interfere with NKG2D activation and/or signaling. The anti-NKG2D antibodies as described herein are also capable of binding portions of NKG2D that interfere with NKG2D ligand binding interaction.

As used herein, an "antigen-binding fragment" of an antibody is a molecule that comprises a portion of a full-length antibody which is capable of detectably binding to the antigen, typically comprising one or more portions of at least the VH region. Antigen-binding fragments include multivalent molecules comprising one, two, three, or more antigen-binding portions of an antibody, and single-chain constructs wherein the VL and VH regions, or selected portions thereof, are joined by synthetic linkers or by recombinant methods to form a functional, antigen-binding molecule. While some antigen-binding fragments of an antibody can be obtained by actual fragmentation of a larger antibody molecule (e.g., enzymatic cleavage), most are typically produced by recombinant techniques.

As used herein, the term "human antibody" is intended to include antibodies having variable regions in which both the framework and CDR regions are derived from (*i.e*., are identical or essentially identical to) human germline immunoglobulin sequences. Furthermore, if the antibody contains a constant region, the constant region also is "derived from" human germline immunoglobulin sequences. The human antibodies of the invention may include amino acid residues not encoded by human germline immunoglobulin sequences (*e.g*., mutations introduced by random or site-specific mutagenesis *in vitro* or by somatic mutation *in viva*). However, the term "human antibody", as used herein, is not intended to include antibodies in which CDR sequences derived from the germline of another mammalian species, such as a mouse, have been grafted onto human framework sequences.

As used herein, a "humanized" antibody is a human/non-human chimeric antibody that contains a minimal sequence derived from non-human immunoglobulin. For the most part, humanized antibodies are human immunoglobulins (recipient antibody) in which residues from a hyper-variable region of the recipient are replaced by residues from a hypervariable region of a non-human species (donor antibody) such as mouse, rat, rabbit, or non-human primate having the desired specificity, affinity, and capacity. In some instances, FR residues of the human immunoglobulin are replaced by corresponding non-human residues. Furthermore, humanized antibodies may comprise residues that are not found in the recipient antibody or in the donor antibody. These modifications are made to further refine antibody performance. In general, a humanized antibody will comprise substantially all of at least one, and typically two, variable domains, in which all or substantially all of the hypervariable loops correspond to those of a non-human immunoglobulin and all or substantially all of the FR residues are those of a human immunoglobulin sequence. The humanized antibody can optionally also comprise at least a portion of an immunoglobulin constant region (Fc), typically that of a human immunoglobulin. For further details, see, e.g., Jones et al., Nature 321:522-525 (1986); Riechmann et al., Nature 332:323-329 (1988); and Presta, Curr. Op. Struct. Biol. 2:593-596 (1992), WO 92/02190, US Patent Application 20060073137, and US Patents 6750325, 6632927, 6639055, 6548640, 6407213, 6180370, 6054297, 5929212, 5895205, 5886152, 5877293, 5869619, 5821337, 5821123, 5770196, 5777085, 5766886, 5714350, 5693762, 5693761, 5530101, 5585089, and 5225539.

As used herein, "framework region" or "FR" residues are those VH or VL residues other than the CDRs as herein defined.

As used herein, an "epitope" or "binding site" is an area or region on an antigen to which an antigen-binding peptide (such as an antibody) specifically binds. A protein epitope may comprise amino acid residues directly involved in the binding (also called the immunodominant component of the epitope) and other amino acid residues, which are not directly involved in the binding, such as amino acid residues which are effectively blocked by the specifically antigen binding peptide (in other words, the amino acid residue is within the "solvent-excluded surface" and/or "footprint" of the specifically antigen binding peptide). The term epitope herein includes both types of amino acid binding sites in any particular region of a hNKG2D that specifically binds to an anti-hNKG2D antibody, or another hNKG2D-specific agent according to the invention, unless otherwise stated (*e.g*., in some contexts the invention relates to antibodies that bind directly to particular amino acid residues). NKG2Ds may comprise a number of different epitopes, which may include, without limitation, (1) linear peptide antigenic determinants, (2) conformational antigenic determinants which consist of one or more non-contiguous amino acids located near each other in a mature NKG2D conformation; and (3) post-translational antigenic determinants which consist, either in whole or part, of molecular structures covalently attached to a NKG2D, such as carbohydrate groups. Unless otherwise specified or contradicted by context, conformational antigenic determinants comprise NKG2D amino acid residues within about 4 A distance from an atom of an antigen-binding peptide.

As used herein, the phrase "binds to essentially the same epitope or determinant as" an antibody of interest (*e.g*., MS or 21 F2) means that an antibody "competes" with the antibody of interest for NKG2D molecules to which the antibody of interest specifically binds.

As used herein, the ability of an anti-NKG2D antibody to "block" the binding of a NKG2D molecule to a natural NKG2D-ligand (*e.g*., MICA), means that the antibody, in an assay using soluble or cell-surface associated NKG2D and ligand molecules, can detectably reduce the binding of a NKG2D-molecule to the ligand in a dose-dependent fashion, where the NKG2D molecule detectably binds to the ligand in the absence of the antibody.

As used herein, the phrase "NKG2D ligand binding interaction" refers to the specific recognition and binding interaction between NKG2D with its ligand (e.g., MICA/B, ULBP/RAET1, Mult1, Rae-1δ, Rae-1ε and H60b). Examples of agents that could modulate the NKG2D ligand binding interaction include soluble NKG2D, an antibody or antigen-binding fragment thereof that specifically binds NKG2D, an NKG2D ligand and an inhibitor of NKG2D ligand activity or expression.

High cholesterol and triglyceride levels are considered to be lipid metabolic disorders that increase risk of cardiovascular diseases and type 2 diabetes.

As used herein, "diabetes" refers to a metabolic disorder that results in elevation of the blood glucose level because of relative or absolute deficiency in the pancreatic hormone insulin. There are two forms of diabetes: type 1 diabetes and type 2 diabetes. Type 1 diabetes is characterized by a progressive loss of pancreatic beta cells due to an unfavourable balance between the destructive autoimmune processes targeting the beta cells on one side and the regenerative capacity of these cells on the other side. This imbalance eventually leads to total loss of beta cells and endogengous insulin secretion. Type 2 diabetes is characterized by insulin resistance and impaired beta cell function, which includes impaired first phase insulin release, reduced beta cell pulse mass and insulin deficiency. In a preferred embodiment, the present invention relates to the treatment of type 2 diabetes.

As used herein, "type 2 diabetes" means a chronic condition in which the body is resistant to the effects of insulin or doesn't produce enough insulin to maintain a normal glucose level. In addition, it also means the metabolic dysfunctions that exist in people in early phases of the disease, such as dyslipidemia.

As used herein, the phrase "cardiovascular disease" is meant abnormal conditions affecting functions associated with the heart and blood vessels.

As used herein, the term "safe and effective amount" refers to the quantity of a component which is sufficient to yield a desired therapeutic response without undue adverse side effects (such as toxicity, irritation, or allergic response) commensurate with a reasonable benefit/risk ratio when used as described herein.

As used herein, the term "therapeutically effective amount" is meant an amount of a composition as described herein effective to yield the desired therapeutic response, for example, an amount effective to delay the development of atherosclerotic plaques, or an amount effective to lower blood glucose levels and promote weight loss in a diabetic patient.

The specific safe and effective amount or therapeutically effective amount will vary with such factors as the particular condition being treated, the physical condition of the patient, the type of mammal or animal being treated, the duration of the treatment, the nature of concurrent therapy (if any), and the specific formulations employed and the structure of the compounds or its derivatives.

As used herein, the term "treatment" is defined as the application or administration of a therapeutic agent to a patient, or application or administration of the therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease, or the predisposition toward disease. For example, "treatment" of a patient in whom no symptoms or clinically relevant manifestations of a disease or disorder have been identified is preventive or prophylactic therapy, whereas clinical, curative, or palliative "treatment" of a patient in whom symptoms or clinically relevant manifestations of a disease or disorder have been identified generally does not constitute preventive or prophylactic therapy. Treatment of type 2 diabetes in a subject, for example, includes lowering blood glucose levels and lowering the subject's body weight. Inhibiting type 2 diabetes disease progression includes preventing or suppressing lipid metabolic conditions, lowering insulin resistance, preventing or suppressing lipid metabolic disorders, improving glucose tolerance, etc. Treating type 2 diabetes and inhibiting type 2 diabetes disease progression can include alleviating or preventing symptoms, disorders or diseases associated with diabetes, e.g., metabolic syndrome, diabetic retinopathy, kidney failure, poor blood circulation and limb disorders associated therewith. Each form of treatment may be considered a distinct aspect of the invention.

Methods involving conventional molecular biology techniques are described herein. Such techniques are generally known in the art and are described in detail in methodology treatises such as Molecular Cloning: A Laboratory Manual, 3rd ed., vol. 1-3, ed. Sambrook et al., Cold Spring Harbor Laboratory Press, Cold Spring Harbor, N.Y., 2001; and Current Protocols in Molecular Biology, ed. Ausubel et al., Greene Publishing and Wiley-Interscience, New York, 1992 (with periodic updates). Conventional methods of gene transfer and gene therapy can also be adapted for use in the present invention. See, e.g., Gene Therapy: Principles and Applications, ed. T. Blackenstein, Springer Verlag, 1999; Gene Therapy Protocols (Methods in Molecular Medicine), ed. P.D. Robbins, Humana Press, 1997; and Retro-vectors for Human Gene Therapy, ed. C.P. Hodgson, Springer Verlag, 1996. Immunology techniques are generally known in the art and are described in detail in methodology treatises such as Advances in Immunology, volume 93, ed. Frederick W. Alt, Academic Press, Burlington, MA, 2007; Making and Using Antibodies: A Practical Handbook, eds. Gary C. Howard and Matthew R. Kaser, CRC Press, Boca Raton, FI, 2006; Medical Immunology, 6th ed., edited by Gabriel Virella, Informa Healthcare Press, London, England, 2007; and Harlow and Lane ANTIBODIES: A Laboratory Manual, Cold Spring Harbor Laboratory Press, Cold Spring Harbor, NY, 1988.

### DETAILED DESCRIPTION OF THE INVENTION

The present invention is based on the discovery that a family of immune stimulatory molecules is upregulated in patients and animals with diabetes or lipid-metabolic dysfunctions and is important for cardiovascular disease development. The data described herein provide the first evidence that MICA/B proteins are elevated in the sera of type 2 diabetic human patients and that injection of anti-NKG2D antibody into diabetic mice blocks the NKG2D ligand interaction and significantly suppresses atherosclerotic plaque formation. The data described herein also shows that NKG2D/ligand mediated immune activation is involved in type 2 diabetic progression. Injection of an anti-NKG2D antibody into an animal model of type 2 diabetes was able to treat diabetes and significantly reduce blood glucose levels. Blocking the NKG2D/ligand interaction also alleviated abnormal metabolic conditions including reducing cholesterol and triglyceride levels.

MICA/B and other NKG2D ligands are therefore novel molecular markers for predicting type 2 diabetes as well as cardiovascular disease development in diabetic patients, and they, as well as the NKG2D/ligand interaction, are novel targets for preventing or treating type 2 diabetes as well as other abnormal metabolic conditions or diseases (e.g., dyslipidemia, diabetic retinopathy, poor blood circulation, limb disorders) and cardiovascular disease (e.g., atherosclerosis).

Accordingly, the present invention provides means for treating type 2 diabetes as well as other abnormal metabolic conditions, such as cardiovascular diseases or disorders.

NKG2D is an immune stimulatory molecule expressed by many immune cells, such as NK, NKT, ab T and gd T cells. Stimulation of NKG2D by its cognate ligands results in activation of the NKG2D-expressing immune cells. There are multiple molecules that could serve as ligands for NKG2D, which include members of Rae-1, H60, and Mult1 in mice and the MICA/B and the ULBP/RAET1 families of proteins in humans. Most of the NKG2D ligands are not expressed or are expressed at low levels in normal cells but are upregulated in various disease conditions, which could in turn activate the NKG2D-expressing immune cells. While such immune activation plays an important role in host defenses such as microbial immunity and tumor surveillance, it could also contribute to immune-mediated diseases.

In one aspect the present disclosure provides methods and kits for detecting MICA/B proteins in a biological sample from a subject to detect the presence of or predict the occurrence of type 2 diabetes, cardiovascular disease, inflammatory disease or a metabolic dysfunction-associated disease in the subject. Metabolic dysfunction-associated diseases include hyperglycemia, diabetic retinopathy, poor blood circulation, limb disorders, and dyslipidemia (e.g., high cholesterol and high triglycerides).

The data described herein in Example 1 indicate that NKG2D ligands are upregulated in type 2 diabetic patients that would promote organ inflammation and associated complications. The data described herein provides that elevated levels of MICA/B proteins were detected in sera and atherosclerotic plaques of type 2 diabetic human patients. It was also found that H60 and Rae-1 (retinoic acid early inducible gene-1) proteins were upregulated in blood vessels especially in atherosclerotic lesion regions, and livers of diabetic or non-diabetic ApoE-/- mice, an animal model for dysfunctional lipid metabolism and atherosclerosis, and that Mult1 was upregulated in the serum of these animals (see Example 2). Thus, the upregulation of NKG2D ligands (e.g., MICA/B proteins or ULBP/RAET1 proteins) may be useful to detect the presence of or predict the occurrence of type 2 diabetes, cardiovascular disease, inflammatory disease or a metabolic dysfunction-associated disease.

A typical method of detecting a predisposition to developing type 2 diabetes, cardiovascular disease, inflammatory disease or a metabolic dysfunction-associated disease or the presence of type 2 diabetes, cardiovascular disease, inflammatory disease or a metabolic dysfunction-associated disease includes obtaining a biological sample from the subject; contacting the biological sample with at least one reagent that detects expression of an NKG2D ligand (e.g., MICA/B expression); measuring the level of expression of the NKG2D ligand in the biological sample; and correlating overexpression of the NKG2D ligand with a predisposition to type 2 diabetes, cardiovascular disease, inflammatory disease or metabolic dysfunction-associated disease development in the subject. As illustrated by the data discussed in Example 1, soluble MICA/B expression was measured. However, in some embodiments, membrane forms of MICA/B expression can be measured. Instead of or in addition to MICA/B, the expression of any NKG2D ligand can be analyzed. The ULBP/RAET1 family of proteins, for example, are NKG2D ligands and have been found to be shed and present in serum, just as MICA/B can be, thus making these proteins suitable markers for the methods, compositions, and kits described herein. NKG2D ligands are described, for example, in Waldhauer, I. and A. Steinle, Oncogene, 27:5932-5943 (2008).

In some embodiments, the reagent that detects expression of an NKG2D ligand (e.g., MICA/B expression) may include any suitable reagent such as MICA/B antibodies and soluble NKG2D.

Generally, the biological sample is serum. Any suitable biological sample, however, can be used. Examples of additional biological samples include biopsy specimens, plasma, urine, skin, blood, and saliva.

Any suitable method or assay can be used to measure the level of NKG2D ligand (e.g., MICA/B) expression in the biological sample of a subject. Numerous antibody-based detection formats are well known in the art, and include ELISA (enzyme linked immunosorbent assay), radioimmunoassays, immunoblots, Western blots, flow cytometry, immunofluorescence assays, immunoprecipitation, protein A assays, immunoelectrophoresis assays, and other related techniques. In some embodiments, antibody binding is detected by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the compositions, kits and methods described herein. Antibodies specific for MICA/B or other NKG2D ligands may be provided in a diagnostic kit that incorporates at least one of these procedures to detect NKG2D ligand (e.g., MICA/B) expression. The kit may contain other components, packaging, instructions, or other material to aid the detection of the protein and use of the kit.

Methods of detecting a predisposition to developing type 2 diabetes, cardiovascular disease, inflammatory disease or a metabolic dysfunction-associated disease or the presence of type 2 diabetes, cardiovascular disease, inflammatory disease or a metabolic dysfunction-associated disease in a subject include correlating overexpression of MICA/B or other NKG2D ligand(s) with a predisposition to type 2 diabetes, cardiovascular disease development, inflammatory disease or a metabolic dysfunction-associated disease or having type 2 diabetes, cardiovascular disease, inflammatory disease or a metabolic dysfunction-associated disease in the subject. Whether or not one or more NKG2D ligands (e.g., MICA/B) are overexpressed in the biological sample can be determined by comparing the level of NKG2D ligand (e.g., MICA/B) expression in the biological sample to a baseline level (also known as a control level) of expression of the NKG2D ligand (e.g., MICA/B). A "baseline level" is a control level, and in some embodiments a normal level or a level not observed in subjects having type 2 diabetes, inflammatory disease or other metabolic dysfunction-associated disease and a predisposition to cardiovascular disease. Therefore, it can be determined, based on the control or baseline level of NKG2D ligand (e.g., MICA/B) expression, whether a sample to be evaluated for type 2 diabetes, cardiovascular disease, inflammatory disease or metabolic dysfunction-associated disease development has a measurable increase (i.e., overexpression, upregulation), decrease, or substantially no change in expression of the NKG2D ligand, as compared to the baseline level. In certain embodiments, the baseline level can be established from a previous sample from the subject being tested, so that the disease state of the subject can be monitored over time and/or so that the efficacy of a given therapeutic protocol can be evaluated over time.

In some aspects of a method of detecting a predisposition to developing type 2 diabetes, cardiovascular disease, inflammatory disease or a metabolic dysfunction-associated disease or the presence of type 2 diabetes, cardiovascular disease, inflammatory disease or a metabolic dysfunction-associated inflammation disease in a subject, expression of one or more markers in addition to an NKG2D ligand (e.g., MICA/B) is analyzed. The one or more markers can also be NKG2D ligands; expression of any NKG2D ligand can be analyzed. Examples of additional markers that can be measured include C-reactive protein, IL-6, TNF-α, sICAM-1, sCD40, the ULBP/RAET1 family of proteins (e.g., ULBP1, ULBP2, ULBP3, ULBP4, RAET1 G, RAET1 L), and others. In embodiments in which expression of one or more markers in addition to an NKG2D ligand (e.g., MICA/B) is analyzed, the combination of the NKG2D ligand (e.g., MICA/B) and other biomarkers may provide a more reliable prediction of vascular disease and/or metabolic dysfunction-associated disease development. In such an embodiment, the biological sample is contacted with at least one reagent that detects the expression of an NKG2D ligand and two or more reagents that detect the presence of expression of the one or more markers in addition to the NKG2D ligand (e.g., MICA/B), respectively. The levels of expression of the NKG2D ligand (e.g., MICA/B) and the two or more additional markers in the biological sample is measured; and overexpression of the NKG2D ligand (e.g., MICA/B) is correlated with a predisposition to type 2 diabetes, cardiovascular disease, inflammatory disease and/or metabolic dysfunction-associated disease (e.g., dyslipidemia, diabetic retinopathy, poor blood circulation, limb disorders, etc.) development in the subject. Depending on the particular two or more additional markers, their underexpression or overexpression may be correlated with a predisposition to type 2 diabetes, cardiovascular disease, inflammatory disease and/or metabolic dysfunction-associated disease development in the subject.

In another embodiment, the present invention provides compositions for modulating NKG2D ligand (e.g., MICA/B or ULBP/RAET1 proteins) expression (e.g., inhibiting MICA/B or ULBP/RAET1 expression) and activity (e.g., binding to NKG2D) to treat a subject having type 2 diabetes, cardiovascular disease, inflammatory disease or a metabolic dysfunction-associated disease. The compositions may also be useful for treating those who are predisposed to and/or has developed cardiovascular disease (e.g., atherosclerosis) and/or who has a metabolic dysfunction-associated disease such as high cholesterol or triglyceride levels, diabetes, dyslipidemia, diabetic retinopathy, poor blood circulation, limb disorders, etc., are described herein. Such compositions generally include a therapeutically effective amount of an agent for modulating expression or activity/signaling of one or more NKG2D ligands (e.g., MICA/B or ULBP/RAET1) and a pharmaceutically acceptable carrier. An inhibitor of an NKG2D ligand (e.g., MICA/B or ULBP/RAET1) is active to reduce the level of NKG2D ligand in a cell and/or to reduce the activity of NKG2D ligand in a cell. An inhibitor of the NKG2D ligand (e.g., MICA/B or ULBP/RAET1), active to reduce the level of an NKG2D ligand in the cell, may be an inhibitor of transcription or translation of the gene encoding the NKG2D ligand. In addition, an inhibitor of an NKG2D ligand active to reduce the level of the NKG2D ligand in the cell may stimulate degradation of the NKG2D ligand and/or NKG2D ligand-encoding RNA. An inhibitor of transcription and/or translation may be a nucleic acid-based inhibitor such as antisense oligonucleotides complementary to a target NKG2D ligand mRNA, as well as ribozymes and DNA enzymes which are catalytically active to cleave the target mRNA.

In some embodiments, a composition described herein includes an siRNA specific for a gene encoding an NKG2D ligand. Sequence-specific siRNA bind to a target nucleic acid molecule, inhibiting the expression thereof. Compositions for delivery of siRNA and methods of treatment thereof are provided. Methods of constructing and using ribozymes, siRNA and antisense molecules are known in the art (e.g., Isaka Y., Curr Opin Mol Ther, 9:132-136 (2007); Sioud M. and Iversen P.O., Curr Drug Targets, 6:647-653 (2005); Ribozymes and siRNA Protocols (Methods in Molecular Biology) by Mouldy Sioud, 2nd ed., 2004, Humana Press, New York, New York). An "antisense" nucleic acid can include a nucleotide sequence which is complementary to a "sense" nucleic acid encoding a protein, e.g., complementary to the coding strand of a double-stranded cDNA molecule or complementary to an mRNA sequence. The antisense nucleic acid can be complementary to an entire MICA/B coding strand, or to only a portion thereof. In another embodiment, the antisense nucleic acid molecule is antisense to a "noncoding region" of the coding strand of a nucleotide sequence encoding an NKG2D ligand (e.g., the 5' and 3' untranslated regions). Anti-sense agents can include, for example, from about 8 to about 80 nucleobases (i.e. from about 8 to about 80 nucleotides), e.g., about 8 to about 50 nucleobases, or about 12 to about 30 nucleobases. Antisense compounds include ribozymes, external guide sequence (EGS) oligonucleotides (oligozymes), and other short catalytic RNAs or catalytic oligonucleotides which hybridize to the target nucleic acid and modulate its expression. Anti-sense compounds can include a stretch of at least eight consecutive nucleobases that are complementary to a sequence in the target gene. An oligonucleotide need not be 100% complementary to its target nucleic acid sequence to be specifically hybridizable. An oligonucleotide is specifically hybridizable when binding of the oligonucleotide to the target interferes with the normal function of the target molecule to cause a loss of utility, and there is a sufficient degree of complementarity to avoid non-specific binding of the oligonucleotide to non-target sequences under conditions in which specific binding is desired, i.e., under physiological conditions in the case of in vivo assays or therapeutic treatment or, in the case of in vitro assays, under conditions in which the assays are conducted.

In some embodiments, a composition for modulating expression of an NKG2D ligand (e.g., inhibiting MICA/B or ULBP/RAET1 expression) and activity (e.g., binding to NKG2D) to treat a subject having a metabolic dysfunction-associated inflammation disease such as type 2 diabetes who is predisposed to and/or has developed cardiovascular disease (e.g., atherosclerosis) and/or who has abnormal metabolic conditions such as high cholesterol or triglyceride levels, dyslipidemia, diabetic retinopathy, etc., includes a therapeutically effective amount of an agent for blocking the interaction of NKG2D with one or more of its ligands (e.g., the NKG2D/MICA/B interaction), a pharmaceutically acceptable carrier, and a known drug for treating cardiovascular disease, high blood pressure, or metabolic disorders that result from a pathway other than the NKG2D/ligand interaction pathway. An example of a known drug for treating cardiovascular disease is any one of the 3-hydroxy-3-methylglutaryl coenzyme A reductase inhibitors (statins). Examples of statins include Cervistatin, Fluvastatin, Atorvastatin, Simvastatin, Pravastatin or Lovastatin, or a pharmaceutically acceptable salt thereof. Compositions and methods involving statins are known in the art (see, e.g., U.S. Patent No. 6,465,454).

In another embodiment, the present invention provides a composition for treating type 2 diabetes, cardiovascular disease, inflammatory disease and/or abnormal metabolic conditions (e.g., high cholesterol levels, high triglyceride levels, dyslipidemia, poor blood circulation, limb disorders, diabetic retinopathy, etc.) that includes an agent for blocking the interaction of NKG2D with one or more of its ligands (e.g., the NKG2D/MICA/B interaction) and a pharmaceutically acceptable carrier. In another embodiment, the composition includes an agent that inhibits NKG2D activation or signaling and a pharmaceutically acceptable carrier.

The data described herein in Example 3 indicate that blocking the NKG2D ligand binding interaction using an anti-NKG2D antibody was able to suppress plaque formation in diabetic ApoE-/- mice without altering the number the NKG2D-expressing cells.

Any suitable agent for blocking the interaction of NKG2D with one or more of its ligands (e.g., the NKG2D/MICA/B interaction) or inhibiting NKG2D activation or signaling can be used. For example, the agent may be selected from the group consisting of soluble NKG2D, an antibody or antigen-binding fragment thereof that specifically binds NKG2D, an NKG2D ligand and an inhibitor of NKG2D ligand activity or expression.

A typical agent that inhibits NKG2D activation or signaling or blocks the interaction of NKG2D with one or more of its ligands is an antibody that specifically binds NKG2D. In some embodiments, the antibody or antigen-binding fragment thereof binds human NKG2D (hNKG2D).

Any suitable antibody that specifically binds to NKG2D can be used. Anti-NKG2D antibodies as described herein include polyclonal and monoclonal human antibodies, or any antigen-binding fragment portions thereof, having at least one antigen binding region of an immunoglobulin variable region, which antibody specifically binds NKG2D. An antibody is specific for NKG2D if it is produced against an epitope of the polypeptide and binds to at least part of the natural or recombinant protein. Another example of an agent that inhibits NKG2D activation or signaling is an antibody that specifically binds an NKG2D ligand (e.g., MICA/B, ULBPs). Any suitable antibody that specifically binds to an NKG2D ligand can be used.

Methods for determining monoclonal antibody specificity and affinity by competitive inhibition can be found in Harlow, et al., Antibodies: A Laboratory Manual, Cold Spring Harbor Laboratory Press, Cold Spring Harbor, N.Y., 1988, Colligan et a/., eds., Current Protocols in Immunology, Greene Publishing Assoc. and Wiley Interscience, N.Y., (1992, 1993), and Muller, Meth. Enzymol. 92:589-601 (1983).

In some embodiments, the antibodies that specifically bind NKG2D are the human monoclonal antibodies 16F16, 16F31, MS, and 21F2 with the sequences described below. In some embodiments, the antibodies are human monoclonal antibodies MS. Full-length, variable, and CDR sequences of these antibodies are set forth in Table 1.

**Table 1**

| **Full-length, variable, and CDR amino acid sequences for 16F16, 16F31, MS, and 21 F2** | | | |
|---|---|---|---|
| **Antibody portion** | **SEQ ID NO:** | **Antibody portion** | **SEQ ID NO:** |
| 16F16 IgG4 H chain | 1 | MS IgG4 H chain | 21 |
| 16F16 L chain | 2 | MS L chain | 22 |
| 16F31 IgG4 H chain | 3 | 21F2 IgG4 H chain | 23 |
| 16F16 L chain | 4 | 21 F2 L chain | 24 |
| 16F16 VH region | 5 | MS VH region | 25 |
| 16F16 VL region | 6 | MS VL region | 26 |
| 16F31 VH region | 7 | 21F2 VH region | 27 |
| 16F31 VL region | 8 | 21F2 VL region | 28 |
| 16F16 VH CDR1 | 9 | MS VH CDR1 | 29 |
| 16F16 VH CDR2 | 10 | MS VH CDR2 | 30 |
| 16F16 VH CDR3 | 11 | MS VH CDR3 | 31 |
| 16F16 VL CDR1 | 12 | MS VL CDR1 | 32 |
| 16F16 VL CDR2 | 13 | MS VL CDR2 | 33 |
| 16F16 VL CDR3 | 14 | MS VL CDR3 | 34 |
| 16F31 VH CDR1 | 15 | 21F2 VH CDR1 | 35 |
| 16F31 VH CDR2 | 16 | 21F2 VH CDR2 | 36 |
| 16F31 VH CDR3 | 17 | 21F2 VH CDR3 | 37 |
| 16F31 VL CDR1 | 18 | 21F2 VL CDR1 | 38 |
| 16F31 VL CDR2 | 19 | 21F2 VL CDR2 | 39 |
| 16F31 VL CDR3 | 20 | 21F2 VL CDR3 | 40 |

Anti-NKG2D and anti-NKG2D ligand antibodies as described herein can be routinely made according to methods such as, but not limited to, inoculation of an appropriate animal with the polypeptide or an antigenic fragment, in vitro stimulation of lymphocyte populations, synthetic methods, hybridomas, and/or recombinant cells expressing nucleic acid encoding such anti-NKG2D antibodies. Immunization of an animal using purified recombinant NKG2D or peptide fragments thereof, is an example of a method of preparing anti-NKG2D antibodies. Similarly, immunization of an animal using purified recombinant NKG2D ligand (e.g., one of the ULBP/RAET1 proteins, MICA/B, Mult1, etc.) or peptide fragments thereof, is an example of a method of preparing anti-NKG2D ligand antibodies.

Monoclonal antibodies that specifically bind NKG2D or an NKG2D ligand may be obtained by methods known to those skilled in the art. See, for example Kohler and Milstein, Nature 256:495-497, 1975; U.S. Pat. No. 4,376,110; Ausubel et al., eds., Current Protocols in Molecular Biology, Greene Publishing Assoc. and Wiley Interscience, N.Y., (1987, 1992); Harlow and Lane ANTIBODIES: A Laboratory Manual Cold Spring Harbor Laboratory Press, Cold Spring Harbor, NY, 1988; Colligan et al., eds., Current Protocols in Immunology, Greene Publishing Assoc. and Wiley Interscience, N.Y., (1992, 1993). Such antibodies may be of any immunoglobulin class including IgG, IgM, IgE, IgA, and any subclass thereof. A hybridoma producing a monoclonal antibody of the present invention may be cultivated in vitro, in situ or in vivo.

In another embodiment, the agent that inhibits NKG2D activation or signaling is a soluble NKG2D. Soluble NKG2D can be prepared by any suitable method known in the art (see, e.g., Diefenbach et al., Nat Immunol. vol. 1(2): 119-26, 2000). In one embodiment, soluble NKG2D is administered to the subject by any suitable delivery route and means. In another embodiment, a nucleic acid encoding soluble NKG2D can be administered to a subject for treating type 2 diabetes, cardiovascular disease, inflammatory disease and/or a metabolic dysfunction-associated disease (e.g., high cholesterol levels, high triglyceride levels, dyslipidemia, poor blood circulation, limb disorders, diabetic retinopathy, etc.). The coding sequence which encodes a soluble NKG2D protein may be identical to the nucleotide sequence of accession no. 574240, or it may also be a different coding sequence which, as a result of the redundancy or degeneracy of the genetic code, encodes the same polypeptide as the polynucleotide of accession no. 574240. Other nucleic acid molecules as described herein include variants of the native NKG2D gene such as those that encode fragments, analogs and derivatives of a native NKG2D protein. Such variants may be, e.g., a naturally occurring allelic variant of the native NKG2D gene, a homolog of the native NKG2D gene, or a non-naturally occurring variant of the native NKG2D gene. These variants have a nucleotide sequence that differs from the native NKG2D gene in one or more bases. For example, the nucleotide sequence of such variants can feature a deletion, addition, or substitution of one or more nucleotides of the native NKG2D gene.

In yet another embodiment, an agent that inhibits NKG2D activation or signaling can be a soluble NKG2D ligand. Shed (soluble) forms of MICA/B have been shown to inhibit NKG2D function throughout the body. In such an embodiment, soluble NKG2D ligand or a nucleic acid encoding a soluble NKG2D ligand can be administered to a subject for treating type 2 diabetes, cardiovascular disease, inflammatory disease and/or a metabolic dysfunction-associated disease (e.g., high cholesterol levels, high triglyceride levels, dyslipidemia, poor blood circulation, limb disorders, diabetic retinopathy, etc.).

In other embodiments, variant NKG2D proteins or NKG2D ligands (e.g., MICA/B, ULBP/RAET1 proteins etc.) displaying substantial changes in structure can be generated by making nucleotide substitutions that cause less than conservative changes in the encoded polypeptide. Examples of such nucleotide substitutions are those that cause changes in (a) the structure of the polypeptide backbone; (b) the charge or hydrophobicity of the polypeptide; or (c) the bulk of an amino acid side chain. Nucleotide substitutions generally expected to produce the greatest changes in protein properties are those that cause non-conservative changes in codons. Examples of codon changes that are likely to cause major changes in protein structure are those that cause substitution of (a) a hydrophilic residue, e.g., serine or threonine, for (or by) a hydrophobic residue, e.g., leucine, isoleucine, phenylalanine, valine or alanine; (b) a cysteine or proline for (or by) any other residue; (c) a residue having an electropositive side chain, e.g., lysine, arginine, or histadine, for (or by) an electronegative residue, e.g., glutamine or aspartine; or (d) a residue having a bulky side chain, e.g., phenylalanine, for (or by) one not having a side chain, e.g., glycine.

Naturally occurring allelic variants of a native Klrk1 gene or native Klrk1 mRNAs as described herein are nucleic acids isolated from human tissue that have at least 75% (e.g., 76%, 77%, 78%, 79%, 80%, 81%, 82%, 83%, 84%, 85%, 86%, 87%, 88%, 89%, 90%, 92%, 93%, 94%, 95%, 96%, 97%, 98%, and 99%) sequence identity with the native Klrk1 gene or native Klrk1 mRNAs, and encode polypeptides having structural similarity to a native Klrk1 protein. Homologs of the native Klrk1 gene or native Klrk1 mRNAs as described herein are nucleic acids isolated from other species that have at least 75% (e.g., 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, and 99%) sequence identity with the native human Klrk1 gene or native human Klrk1 mRNAs, and encode polypeptides having structural similarity to native human NKG2D protein. Public and/or proprietary nucleic acid databases can be searched to identify other nucleic acid molecules having a high percent (e.g., 70, 80, 90% or more) sequence identity to the native Klrk1 gene or native Klrk1 mRNAs.

Non-naturally occurring Klrk1 gene or mRNA variants are nucleic acids that do not occur in nature (e.g., are made by the hand of man), have at least 75% (e.g., 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, and 99%) sequence identity with the native human Klrk1 gene or native human Klrk1 mRNAs, and encode polypeptides having structural similarity to native human NKG2D protein. Examples of non-naturally occurring Klrk1 gene variants are those that encode a fragment of a NKG2D protein, those that hybridize to the native Klrk1 gene or a complement of the native Klrk1 gene under stringent conditions, those that share at least 65% sequence identity with the native Klrk1 gene or a complement thereof, and those that encode a NKG2D fusion protein.

Nucleic acids encoding fragments of a native NKG2D protein as described herein are those that encode, e.g., 2, 5, 10, 25, 50, 100, 150, 200 or more amino acid residues of the native NKG2D protein. Shorter oligonucleotides (e.g., those of 6, 7, 8, 9, 10, 11, 12, 13, 14, 15, 16, 17, 18, 19, 20, 30, 50, base pairs in length) that encode or hybridize with nucleic acids that encode fragments of a native NKG2D protein can be used as probes, primers, or antisense molecules. Nucleic acids encoding fragments of a native NKG2D protein can be made by enzymatic digestion (e.g., using a restriction enzyme) or chemical degradation of the full length native Klrk1 gene, a Klrk1 mRNA or cDNA, or variants of the foregoing. Using the nucleotide sequence of the native human Klrk1 gene and the amino acid sequence of the native NKG2D protein previously reported, those skilled in the art can create nucleic acid molecules that have minor variations in their nucleotide sequence, by, for example, standard nucleic acid mutagenesis techniques or by chemical synthesis. Variant Klrk1 nucleic acid molecules can be expressed to produce variant NKG2D proteins.

In some embodiments, a composition for inhibiting type 2 diabetes, cardiovascular disease, inflammatory disease and/or treating abnormal metabolic conditions (e.g., high cholesterol levels, high triglyceride levels, dyslipidemia, diabetic retinopathy, limb disorders, poor blood circulation, etc.) includes an agent (e.g., anti-NKG2D antibody or anti-NKG2D ligand antibody) for blocking the interaction of NKG2D with one or more of its ligands (e.g., the NKG2D/MICA/B interaction), a pharmaceutically acceptable carrier, and a known drug for treating cardiovascular disease. An example of a known drug for treating cardiovascular disease is any one of the 3-hydroxy-3-methylglutaryl coenzyme A reductase inhibitors (statins). Examples of statins include Cervistatin, Fluvastatin, Atorvastatin, Simvastatin, Pravastatin or Lovastatin, or a pharmaceutically acceptable salt thereof. Compositions and methods involving statins are known in the art (see, e.g., U.S. Patent No. 6,465,454).

The data described herein in Example 3 illustrates that blocking the NKG2D ligand binding interaction using an anti-NKG2D antibody significantly suppressed the atherosclerotic plaque formation in diabetic ApoE-/- mice, suggesting that the NKG2D/ligand interaction is a critical pathway in promoting cardiovascular disease development and can serve as a drug target for the prevention or treatment of cardiovascular disease. The data described herein in Examples 3 and 4 further illustrates the critical role of NKG2D/ligand interaction in atherosclerosis and inflammation using NKG2D knockout mice and anti-NKG2D antibody studies. Blocking the NKG2D/ligand interaction also alleviated abnormal metabolic conditions including reducing cholesterol and triglyceride levels as illustrated in Example 5. Further comparison of cytokine expression profiles in the anti-NKG2D antibody treated and control groups demonstrated that the anti-NKG2D antibody treatment significantly suppressed the production of multiple pro-inflammatory cytokines, suggesting that it functions by preventing vascular inflammation (see Example 4). The data described herein also provides that the NKG2D/ligand mediated immune activation is involved in type 2 diabetic progression. In the experiments described herein in Example 7, compared to the Western diet fed ApoE-/- mice, the NKG2D-deficient ApoE-/-Klrk1-/- mice fed with the same diet had significantly lower glucose levels in the blood, demonstrating the usefulness of targeting NKG2D or its ligands to treat type 2 diabetes. Moreover, anti-NKG2D antibody treatment was able to prevent the development and progression of hyperglycaemia and diabetes in a rodent model of type 2 diabetes, as described in Example 8.

In another embodiment, the present invention provides for treating type 2 diabetes, cardiovascular disease, inflammatory disease and/or abnormal metabolic conditions (e.g., high cholesterol levels, high triglyceride levels, dyslipidemia, diabetic retinopathy, limb disorders, poor blood circulation, etc.) in a subject. In certain embodiments, the present invention provides for treating type 2 diabetes, In another embodiment, the present invention provides for treating a condition that may be regulated or normalised via inhibition of NKG2D in a subject. Such conditions include, for example, type 2 diabetes, cardiovascular disease, inflammatory disease and/or abnormal metabolic conditions (e.g., high cholesterol levels, high triglyceride levels, dyslipidemia, diabetic retinopathy, limb disorders, poor blood circulation, etc.). In certain embodiments, the condition is type 2 diabetes. In certain other embodiments, the condition is cardiovascular disease.

A typical treatment includes administering a therapeutically effective amount of an agent that inhibits NKG2D activation or signaling for inhibiting type 2 diabetes, cardiovascular disease, inflammatory disease and/or abnormal metabolic conditions and a pharmaceutically acceptable carrier to the subject. In certain embodiments, it includes administering a therapeutically effective amount of an agent that blocks the NKG2D ligand binding interaction. The agent can be any of those described above, e.g., soluble NKG2D, antibody or antigen-binding fragment thereof that specifically binds NKG2D, soluble NKG2D ligand, antibody specific for an NKG2D ligand, an inhibitor of NKG2D ligand (e.g., MICA/B) activity or expression. In certain embodiments, the agent is an antibody or antigen-binding fragment thereof that specifically binds NKG2D. In certain embodiments, the antibody or antigen-binding fragment thereof is human or humanized. In certain embodiments, the antibody or antigen-binding fragment thereof binds human NKG2D (hNKG2D). In certain embodiments, the antibody or antigen-binding fragment thereof reduces NKG2D-mediated activation or signalling of an NKG2D-expressing cell. In certain embodiments, the antibody or antigen-binding fragment thereof competes with at least one NKG2D ligand in binding to NKG2D. In certain embodiments, the NKG2D ligand is MICA/B.

In certain embodiments, administration of the agent results in at least one of the following in the subject: reduces blood glucose levels, improves glucose tolerance, lowers insulin resistance, reduces body weight, lowers blood pressure, lowers inflammation or reduces metabolic dysfunctions.

The therapies described herein (which include prophylactic treatment) in general include administration of a therapeutically effective amount of the compositions described herein to a subject (e.g., animal, human) in need thereof, including a mammal, particularly a human. Such treatment will be suitably administered to subjects, particularly humans, suffering from, having, susceptible to, or at risk for a disease, disorder, or symptom thereof. Determination of those subjects "at risk" can be made by any objective or subjective determination by a diagnostic test or opinion of a subject or health care provider (e.g., genetic test, enzyme or protein marker, marker (as defined herein), family history, and the like). The compositions herein may be also used in the treatment of any other disorders in which an excess of NKG2D signaling, expression, or activity may be implicated.

In one embodiment, a method of treating type 2 diabetes, cardiovascular disease, inflammatory disease and/or abnormal metabolic conditions (e.g., high cholesterol levels, high triglyceride levels, dyslipidemia, diabetic retinopathy, limb disorders, poor blood circulation, etc.) in a subject includes monitoring treatment progress. One can monitor treatment progress by determining a level of diagnostic marker (e.g., any target delineated herein modulated by a composition or agent described herein, a protein or indicator thereof, etc.) or diagnostic measurement (e.g., screen, assay) in a subject suffering from or susceptible to a disorder or symptoms thereof associated with type 2 diabetes, cardiovascular disease, inflammatory disease and/or abnormal metabolic conditions (e.g., high cholesterol levels, high triglyceride levels, dyslipidemia, diabetic retinopathy, limb disorders, poor blood circulation, etc.) in which the subject has been administered a therapeutic amount of a composition as described herein sufficient to treat the disease or symptoms thereof. The level of marker determined in the method can be compared to known levels of marker in either healthy normal controls or in other afflicted patients to establish the subject's disease status. Generally, a second level of marker in the subject is determined at a time point later than the determination of the first level, and the two levels are compared to monitor the course of disease or the efficacy of the therapy. In certain embodiments, a pre-treatment level of marker in the subject is determined prior to beginning treatment as described herein; this pre-treatment level of marker can then be compared to the level of marker in the subject after the treatment commences, to determine the efficacy of the treatment.

The administration of compositions including an inhibitor of NKG2D ligand activity or expression (e.g., a MICA/B inhibitor), soluble NKG2D, soluble NKG2D ligand(s), anti-NKG2D antibody, anti-NKG2D ligand antibody, etc. for the treatment of diabetes, cardiovascular disease (e.g., atherosclerosis) and/or abnormal metabolic conditions (e.g., high cholesterol levels, high triglyceride levels, dyslipidemia, diabetic retinopathy, limb disorders, poor blood circulation, etc.) may be by any suitable means that results in a concentration of the therapeutic that, combined with other components, is effective in ameliorating, reducing, or eliminating type 2 diabetes, cardiovascular disease (e.g., suppress atherosclerotic plaque formation), inflammatory disease and/or abnormal metabolic conditions (e.g., type high cholesterol levels, high triglyceride levels, dyslipidemia, diabetic retinopathy, limb disorders, poor blood circulation, etc.). The inhibitor of NKG2D ligand activity or expression (e.g., a MICA/B inhibitor), soluble NKG2D, soluble NKG2D ligand(s), anti-NKG2D antibody, anti-NKG2D ligand antibody, etc., may be contained in any appropriate amount in any suitable carrier substance, and is generally present in an amount of 1-95% by weight of the total weight of the composition. The composition may be provided in a dosage form that is suitable for local or systemic administration (e.g., parenteral, subcutaneously, intravenously, intramuscularly, or intraperitoneally). In certain embodiments, the composition is administered intravenously, intraperitoneally or subcutaneously. The pharmaceutical compositions may be formulated according to conventional pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York).

Compositions as described herein may be administered parenterally by injection, infusion or implantation (subcutaneous, intravenous, intramuscular, intraperitoneal, or the like) in dosage forms, formulations, or via suitable delivery devices or implants containing conventional, non-toxic pharmaceutically acceptable carriers and adjuvants. The formulation and preparation of such compositions are well known to those skilled in the art of pharmaceutical formulation. Formulations can be found in Remington: The Science and Practice of Pharmacy, supra.

Compositions for parenteral use may be provided in unit dosage forms (e.g., in single-dose ampules), or in vials containing several doses and in which a suitable preservative may be added (see below). The composition may be in the form of a solution, a suspension, an emulsion, an infusion device, or a delivery device for implantation, or it may be presented as a dry powder to be reconstituted with water or another suitable vehicle before use. Apart from the active agent, the composition may include suitable parenterally acceptable carriers and/or excipients. The active therapeutic agent(s) may be incorporated into microspheres, microcapsules, nanoparticles, liposomes, or the like for controlled release. Furthermore, the composition may include suspending, solubilizing, stabilizing, pH-adjusting agents, tonicity adjusting agents, and/or dispersing agents.

As indicated above, the pharmaceutical compositions according to the invention may be in the form suitable for sterile injection. To prepare such a composition, the suitable active therapeutic(s) are dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution, and isotonic sodium chloride solution and dextrose solution. The aqueous formulation may also contain one or more preservatives (e.g., methyl, ethyl or n-propyl p-hydroxybenzoate). In cases where one of the compounds is only sparingly or slightly soluble in water, a dissolution enhancing or solubilizing agent can be added, or the solvent may include 10-60% w/w of propylene glycol or the like.

Materials for use in the preparation of microspheres and/or microcapsules are, e.g., biodegradable/bioerodible polymers such as polygalactin, poly-(isobutyl cyanoacrylate), poly(2-hydroxyethyl-L-glutam- nine) and, poly(lactic acid). Biocompatible carriers that may be used when formulating a controlled release parenteral formulation are carbohydrates (e.g., dextrans), proteins (e.g., albumin), lipoproteins, or antibodies. Materials for use in implants can be non-biodegradable (e.g., polydimethyl siloxane) or biodegradable (e.g., poly(caprolactone), poly(lactic acid), poly(glycolic acid) or poly(ortho esters) or combinations thereof).

At least two anti-cardiovascular disease therapeutics (e.g., NKG2D or a MICA/B inhibitor and a statin) may be mixed together in one formulation.

Compositions as described herein may also be combined with a second or more pharmacologically active agent, e.g., selected from antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. Examples of these pharmacologically active substances are: GLP-1 and GLP-1 derivatives and analogues, GLP-2 and GLP-2 derivatives and analogues, Exendin-4 and Exendin-4 derivatives and analogues, amylin and amylin derivatives and analogues, sulphonylureas, biguanides, meglitinides, glucosidase inhibitors, glucagon antagonists, DPP-IV (dipeptidyl peptidase-IV) inhibitors, SGLT2 inhibitors, SGLT1 inhibitors, inhibitors of hepatic enzymes involved in stimulation of gluconeogenesis and/or glycogenolysis, glucose uptake modulators, compounds modifying the lipid metabolism such as antihyperlipidemic agents as HMG CoA inhibitors (statins), compounds lowering food intake, RXR agonists and agents acting on the ATP-dependent potassium channel of the β-cells; Cholestyramine, colestipol, clofibrate, gemfibrozil, lovastatin, pravastatin, simvastatin, probucol, dextrothyroxine, nateglinide, repaglinide; β-blockers such as alprenolol, atenolol, timolol, pindolol, propranolol and metoprolol, ACE (angiotensin converting enzyme) inhibitors such as benazepril, captopril, enalapril, fosinopril, lisinopril, alatriopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin; CART (cocaine amphetamine regulated transcript) agonists, NPY (neuropeptide Y) antagonists, PYY (polypeptide YY) agonists, PP (pancreatic polypeptide) agonists, Y2 receptor agonists, Y4 receptor agonits, mixed Y2/Y4 receptor agonists, adiponectin agonists, PPAR agonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, TR β agonists; histamine H3 antagonists, gastrin and gastrin analogues and derivatives, glucagon and glucagon derivatives and analogues, FGF-21 (fibroblast growth factor 21) and FGF-21 derivatives and analogues, proton pump inhibitors such as lansoprazole, omeprazole, dexlansoprazole, esomeprazole, pantoprazole, and rabeprazole and glucokinase activators.

It should be understood that any suitable combination of the compositions according to the invention with one or more of the above-mentioned compounds and optionally one or more further pharmacologically active substances are considered to be within the scope of the present invention.

Although many of the compositions, methods and kits described above pertain to treating type 2 diabetes and cardiovascular disease, the compositions and methods described herein can also be used to prevent any metabolic dysfunction-associated disease in a subject, including type 2 diabetes and cardiovascular diseases. In certain embodiments, the compositions and methods of the present invention can prevent or abolish the symptoms of a disease, a delay in onset of the symptoms of a disease, or lessening in the severity of a disease.

The compositions and methods described herein can be used to detect, prevent and/or treat any metabolic dysfunction-associated disease in a subject, regardless of whether or not the subject has type 2 diabetes. For example, a method of detecting elevated levels of cholesterol and triglycerides in a subject is described herein and includes obtaining a biological sample from the subject, contacting the biological sample with at least one reagent that detects expression of an NKG2D ligand (e.g., MICA/B expression); measuring the level of expression of the NKG2D ligand in the biological sample; and correlating overexpression of the NKG2D ligand (e.g., MICA/B) with a predisposition to having elevated levels of cholesterol and triglycerides or the presence of elevated levels of cholesterol and triglycerides in the subject. Treating a subject (who may or may not have type 2 diabetes) having elevated levels of cholesterol and triglycerides includes administering a therapeutically effective amount of an agent that inhibits NKG2D activation or signaling for lowering cholesterol and triglyceride levels and a pharmaceutically acceptable carrier to the subject. As another example, described herein is the treatment of a subject having diabetic retinopathy. Such treatment includes administering a therapeutically effective amount of an agent that inhibits NKG2D activation or signaling for inhibiting diabetic retinopathy and a pharmaceutically acceptable carrier to the subject. The agent that inhibits NKG2D activation or signaling for inhibiting diabetic retinopathy or lowering cholesterol and triglyceride levels can be any of those described above, e.g., soluble NKG2D, antibody specific for NKG2D, soluble NKG2D ligand, antibody specific for an NKG2D ligand, an inhibitor of NKG2D ligand (e.g., MICA/B) activity or expression, etc. The agent and pharmaceutically acceptable carrier can be packaged in a kit as described above.

In additional embodiments, compositions and methods for treating type 2 diabetes, cardiovascular disease, inflammatory disease and/or metabolic dysfunction-associated diseases (e.g., high cholesterol levels, high triglyceride levels, dyslipidemia, poor blood circulation, limb disorders, diabetic retinopathy, etc.) can include an agent that promotes shedding of an NKG2D ligand from the surface of cells. NKG2D ligands are often cleaved from the cell surface by proteases (called "shedding") and shedding is generally thought to inhibit NKG2D signaling by binding of the shed ligand to NKG2D and downregulating NKG2D activity (e.g., signaling). Any agent that promotes or inhibits shedding can modulate NKG2D signalling and activity and can be used in such an embodiment. For example, an antibody or antigen binding fragment against a protein involved in shedding can be administered to a subject in an amount effective for promoting shedding of at least one NKG2D ligand.

Additionally, the compositions, kits and methods described herein can be used to prevent a metabolic dysfunction-associated disease in a subject. For example, a physician may administer the compositions and methods described herein to a subject who has a predisposition to cardiovascular disease and/or a metabolic dysfunction-associated disease, whether due to a family history (genetics) or environmental factors (e.g., diet, lack of exercise, etc.).

The compositions described above are preferably administered to a subject (e.g., human) in an effective amount, that is, an amount capable of producing a desirable result in the treated subject (e.g., inhibiting or preventing type 2 diabetes, cardiovascular disease such as atherosclerosis, lowering blood glucose levels and promoting weight loss in a type 2 diabetes patient, reducing cholesterol or triglyceride levels in the subject, lowering insulin resistance in the subject, preventing or alleviating dyslipidemia, diabetic retinopathy, limb disorders, poor blood circulation, etc., in the subject). Toxicity and therapeutic efficacy of the compositions described herein can be determined by standard pharmaceutical procedures. As is well known in the medical and veterinary arts, dosage for any one animal depends on many factors, including the subject's size, body surface area, age, the particular composition to be administered, time and route of administration, general health, and other drugs being administered concurrently.

The amount of the therapeutic agent to be administered varies depending upon the manner of administration, the age and body weight of the patient, and with the clinical symptoms of the cancer. A composition as described herein is typically administered at a dosage that inhibits NKG2D activity and/or signaling, as assayed by identifying a reduction in atherosclerotic plaque formation, blood glucose levels, body weight, cholesterol levels, triglyceride levels, insulin resistance, etc., or using any that assay that measures the expression or the biological activity of an NKG2D ligand (e.g., MICA/B or ULBP/RAET1) or NKG2D activity or signaling.

Described herein are kits for detecting NKG2D ligands (e.g., MICA/B or ULBP/RAET1 proteins) in a biological sample from a subject (e.g., human) to detect the presence of or predict the occurrence of type 2 diabetes, cardiovascular disease, inflammatory disease and/or abnormal metabolic conditions (e.g., high cholesterol levels, high triglyceride levels, dyslipidemia, diabetic retinopathy, limb disorders, poor blood circulation, etc.) in the subject. A typical kit includes at least one reagent for detecting the expression of an NKG2D ligand (e.g., MICA/B or ULBP/RAET1) in a biological sample from the subject and instructions for use. In one embodiment, a kit includes a monoclonal or polyclonal antibody to a ULBP/RAET1 protein, or to MICA/B, a detectable label, and instructions for use. Generally, the biological sample is serum. However, any suitable biological sample can be used. Examples of additional biological samples include blood, plasma, urine, saliva, skin, and biopsy samples.

Kits for administering treatment to a subject (e.g., human) having diabetes or any conditions that may be regulated or normalised via inhibition of NKG2D, such as type 2 diabetes, cardiovascular disease, inflammatory disease and/or abnormal metabolic conditions (e.g., high cholesterol levels, high triglyceride levels, dyslipidemia, diabetic retinopathy, limb disorders, poor blood circulation, etc.) are also described herein. In one embodiment, the kit includes a therapeutic or prophylactic composition containing a therapeutically effective amount of an agent for blocking the interaction of NKG2D and one or more of its ligands (e.g., the NKG2D/MICA/B interaction, the interaction of NKG2D and one of the ULBP/RAET1 proteins, etc.) or for inhibiting NKG2D activation or signaling and a pharmaceutically acceptable carrier in unit dosage form. The agent may be a soluble NKG2D, an antibody or antigen-binding fragment thereof that specifically binds NKG2D, an NKG2D ligand and an inhibitor of NKG2D ligand activity or expression. In certain embodiments, the agent is an antibody or antigen-binding fragment thereof that specifically binds NKG2D. In certain embodiments, the antibody or antigen-binding fragment thereof is human or humanized. In certain embodiments, the antibody or antigen-binding fragment thereof binds human NKG2D (hNKG2D).

If desired, the kit also contains an effective amount of a known drug for treating cardiovascular disease (e.g., a statin) and/or a drug for treating an abnormal metabolic condition (e.g., high cholesterol levels, high triglyceride levels, dyslipidemia, diabetic retinopathy, limb disorders, poor blood circulation, etc.).

In certain embodiments, the kit further comprises at least one additional agent selected from the group consisting of antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity.

Generally, a kit as described herein includes packaging and instructions for use. In some embodiments, the kit includes a sterile container which contains a therapeutic or prophylactic composition; such containers can be boxes, ampules, bottles, vials, tubes, bags, pouches, blister-packs, or other suitable container forms known in the art. Such containers can be made of plastic, glass, laminated paper, metal foil, or other materials suitable for holding medicaments.

The present invention is further illustrated by the following specific examples.

### EXAMPLES

### EXAMPLE 1: Detection of MICA, a ligand for NKG2D, in sera and atherosclerotic plaques of type 2 diabetes patients and its association with expression of other proinflammatory cytokines

To determine whether metabolic dysfunction might cause upregulation of certain stress response immune stimulating molecules, MICA/B expression was assessed in a group of type 2 diabetic patients who have confirmed diabetes and, in some, have dyslipidemia as well. Type 1 diabetic patients were not included because they are mostly of autoimmune diseases and might have MICA upregulation independent of the metabolic conditions, as inferred from type 1 diabetic NOD mouse studies (Ogasawara et al., Immunity, 20:757-767 (2004)). Since it is unpractical to analyze MICA/B expression on blood vessels of the patients, levels of soluble MICA (sMICA) proteins in the blood were assayed. The sMICA is enzymatically cleaved products of membrane MICA (mMICA) expressed on the cell surface and was previously reported to be detected in the blood of various cancer patients and immune disease patients but not in healthy people. Therefore, the detection of sMICA in the blood of the diabetic patients would indicate MICA upregulation in the blood vessels or other tissues.

Twenty-two patients were tested and elevated levels of sMICA were detected in significant percentages of them (Fig. 1A). Of the 22 patients, six (27%) had serum MICA at levels of higher than 400 pg/ml with the highest at 12250 pg/ml (Fig. 1A, Patient #1-6). In addition, four patients (18%) had detectable levels of serum sMICA (50-350 pg/ml) (Fig. 1A, patients #7-10). To determine how the sMICA levels in the diabetic patients were comparable to those in cancer patients, 50 serum samples from various cancer patients were also tested. The type 2 diabetic patients have more significantly elevated levels of sMICA proteins than the cancer patients since the sMICA levels of the 50 cancer patients were all below 20 pg/ml (Fig. 1 E). Although the sMICA levels in cancer patients vary a lot depending on cancer types and stages, those of the type 2 diabetic patients were still at the higher end when compared to the sMICA levels of cancer patients reported in the literature (Salih et al., J Immunol., 169:4098-4102 (2002); Groh et al., Nature, 419:734-738 (2002)).

Considering that these patients have been diagnosed with diabetes for 1 to 20 years with various healthy conditions, it is not surprising that they have variable levels of sMICA. In spite of the variation, certain trends were found that were associated with the high serum sMICA level, especially when comparing the MICA-high with MICA-negative patients. Particularly, the average blood glucose level was higher in the MICA-high patients than in the MICA-negative patients (190±65 vs. 157±47; p = 0.1). The high levels of sMICA proteins in the blood indicated that membrane MICA was expressed on certain cell types that, if on arteries, may impact vascular inflammation through interaction with its receptor NKG2D expressed on the multiple immune cells. It should be noted that although both membrane and soluble forms of MICA proteins bind to NKG2D, they have different effects on immune activation. NKG2D/mMICA engagement activates the immune cells while NKG2D/sMICA interaction does not. In fact, sMICA might interfere with NKG2D/mMICA interaction-mediated immune activation through the competition with mMICA for the NKG2D binding. Therefore, expression of several inflammation-associated cytokines and factors in the sera of the diabetic patients was analyzed and an attempt to correlate them with soluble MICA expression was made.

Levels of cytokines IL-6, TNF-α, IL-1β, interferon-γ (IFN-γ) and C-reactive protein (CRP) in the sera of the same diabetic patients was assessed to determine whether upregulation of sMICA is associated with any of these parameters (Fig. 1B-D). The IL-1β and IFN-γ were below detectable range of the assays. There was no correlation between levels of sMICA/B and TNF-α or CRP (Fig. 1A, C&D), suggesting that MICA expression was regulated differentially from the TNF-α or CRP, two well-defined inflammation markers. Interestingly, there was an inverse correlation between sMICA/B and IL-6 expression in the sera (Fig. 1A & B). The serum level of IL-6 in MICA-positive group (patients #1-10) was significantly lower than that of MICA-negative group (patients #11-22) (1.21±0.51 ng/ml vs. 1.85±1.13 ng/ml, P=0.05), suggesting an interdependent relationship between these two factors.

Together, these data demonstrated that MICA/B molecules are upregulated in the type 2 diabetic patients and might be involved in vascular inflammation and atherosclerosis. Although a high level of sMICA clearly indicates the MICA upregulation in the diabetic patients, the net effect of MICA upregulation on inflammation and atherosclerosis depends on relative contribution of the soluble and membrane MICA proteins. Generation of sMICA is usually a compensation mechanism for mMICA/NKG2D interaction to regulate the immune activation. Therefore, mouse models were used to directly assess the upregulation of NKG2D ligands in arteries and its importance in the inflammation and atherosclerosis.

To further establish clinical relevance of the NKG2D ligand expression, immunohistochemical staining of atherosclerotic aortic sections of human patients was performed to determine whether the ligands of NKG2D were upregulated in the tissues. In contrast to plaque-free aortae (Fig. 2I), many cells in the atherosclerotic aortae stained positive with a MICA/B antibody (Fig. 2A, C, E and G). The overlapping staining patterns of MICA/B with Mac-3 or CD31 in adjacent sections suggested that both macrophages and endothelial cells of the atherosclerotic aortae expressed MICA/ B (Fig. 2B, D, F, H), suggesting that human have similar expression patterns of NKG2D ligands in affected tissues.

### EXAMPLE 2: Upregulation of NKG2D ligands in cells of atherosclerotic plaques and other tissues of ApoE-/- mice

To test directly whether the ligands for NKG2D were upregulated in blood vessels of diabetic and dyslipidemic mice, expression of NKG2D ligands was determined in ApoE-/- mice, an animal model of dyslipidemia and atherosclerosis. It has been well established that the chronic dysfunctional metabolism in the ApoE-/- mice leads to the development of atherosclerotic plaques, in which immune cells account for a significant portion. These mice have impaired lipid metabolism and develop atherosclerosis as they age. The upregulation of NKG2D ligands in aortae of ApoE-null mice that developed atherosclerotic plaques was analyzed.

First, mRNA expression of the NKG2D ligands was examined in aortae of 8-10 month-old ApoE-/- mice (no diabetic induction) which had atherosclerotic plaques in the aorta, especially in the arch areas. Referring to Fig. 3A, to determine specifically whether the plaque lesions have different levels of NKG2D ligand expression, RNAs were isolated separately from the atherosclerotic aortic arch ("plaqued") and the thoracic part of an aorta that did not have apparent plaques ("plaque-free") of 8-10 month-old, non-diabetic ApoE-/- mice ("Atherosclerotic ApoE-/-"). RNAs of corresponding parts of an aorta from 2 month-old, plaque-free ApoE-/- mice were used as controls ("plaque-free ApoE-/-"). To more specifically determine upregulation of the different individual NKG2D ligands, isoform-specific primers for the different isoforms of Rae-1 genes, as well as H60 and Mult-1 genes, were used. Rae-1, H60 and MULT-1 are all NKG2D ligands that belong to different families (Diefenbach et al., Nature Immunology, 1:119-126 (2000); Takada et al., J Immunol., 180:1678-1685 (2008)). There are five closely related Rae-1 genes in mice (>98% identical) and three different isoforms of H60 genes (H60a, b and c) have been identified (Diefenbach et al., Nature Immunology, 1:119-126 (2000); Takada et al., J Immunol., 180:1678-1685 (2008)). The transcripts for Rae-1δ, Rae-1ε and H60 were analyzed by semi-quantitative radioactive RT-PCR. Briefly, The RNA samples were reverse transcribed with Superscript II RNase-H reverse transcriptase using oligo-dT primers. The RT products were serially diluted (5-fold each) and subjected to PCR using gene-specific primers in the presence of P³² dCTP. The primers used were based on reported gene sequences (Diefenbach et al., Nature Immunology, 1:119-126 (2000); Takada et al., J Immunol., 180:1678-1685 (2008)). The PCR products were run on a polyacrylamide gel, which was then dried and exposed to a Phospho-Image screen. β-Actin was used as loading control.

Compared to those in the controls, transcripts for Rae-1δ, Rae-1ε and H60b were drastically upregulated in the plaque regions of the atherosclerotic ApoE-null mice (Fig. 3A). Compared to the atherosclerosis-free younger ApoE-null controls, up to 100-fold increases of Rae-1δ, Rae-1ε and H60b transcripts were detected in the plaque areas (Fig. 3A). Even when compared to the non-plaque part of the aorta of the same mouse, 10- 20 fold increases of the Rae-1δ, Rae-1ε and H60β genes were found in the plaque area (Fig. 3A). The non-plaque region of the aorta of the old ApoE-/- mice had modest upregulation of Rae-1δ&ε when compared to the corresponding region of the young atherosclerosis-free ApoE-/- mice. Much higher levels of transcripts for NKG2D ligands Rae-1δ, Rae-1ε and H60b (10-30 fold increase) were also detected in aortic arches of Western diet (WD)-fed ApoE^{-/-} mice than in age-matched wild type (WT) controls (Fig. 3B). These data suggested that certain cellular components of the atherosclerotic plaques have very dramatic upregulation of the NKG2D ligand expression.

To further confirm the upregulation of the NKG2D ligands in the plaques, immunohistochemical staining was performed on the cryosections of atherosclerotic plaques of the ApoE-null mice with polyclonal anti-Rae-1 and H60 antibodies. Since diabetes and high fat Western diets accelerate plaque formation and might be involved in upregulation of the NKG2D ligands, plaques in ApoE-null mice that were rendered diabetic by streptozotocin (STZ) injection or fed with a Western diet were also examined. Referring to Fig. 3C, for the STZ treatment, six-week-old ApoE-null mice (Jackson Lab, Bar Harbor, Maine) were induced to develop diabetes by intraperitoneal injection of streptozotocin (STZ) (55 mg/kg, freshly dissolved in pH 4.5 citrate buffer) for six consecutive days and confirmed by blood glucose concentration (>300 mg/dL). Two months after the diabetic induction, aortae were isolated and cryopreserved. The cryosections were stained with polyclonal goat anti-Rae-1 or H60 antibody (R&D Systems and Santa Cruz Biotechnology respectively), followed by horseradish peroxidase (HRP)-conjugated donkey anti-goat IgG antibody and ABC-HRP staining system to reveal positive staining (brown color) (Santa Cruz Biotechnology). The sections were further stained with monoclonal rat anti-mouse CD68 antibody (Serotec), followed by Alkaline Phosphotase (AP)-conjugated donkey anti-rat antibody and ABC-AP staining kit (blue color) (Vector Laboratory). For the Western diet fed ApoE-/- model of atherosclerosis, six-week-old ApoE-null mice switched from normal chow to the Western diet. Two months after being on the Western diet, aortae were isolated and cryopreserved and analyzed by anti-Rae-1 and H60 antibody staining (Fig. 3D).

Positive staining for both Rae-1 and H60 were apparent in the sections of the plaque areas but much less in tunica media or adventitia of the atherosclerotic aortic arch regions of both diabetic and non-diabetic ApoE null mice (Fig. 3C and 3D). As controls, nearly not Rae-1 or H60 staining was detected in plaque-free aortic regions of the same mice (Fig. 3D, bottom panels). Staining for CD68, a marker for tissue resident macrophages, had a significant overlap with the RAE-1 and H60 staining, indicating that the plaque-infiltrating macrophages were at least one cellular population of the plaques that expressed the NKG2D ligands, which was further confirmed by flow cytometric analysis of Rae-1 expression on macrophages directly isolated from atherosclerotic aortae (Fig. 3E). In addition, endothelial cells isolated from aortae of the atherosclerotic ApoE-/- mice also had higher Rae-1 expression (Fig. 3E).

Considering their defective metabolism, macrophages of ApoE-/- mice likely upregulated the NKG2D ligand expression in response to increased abnormal metabolites in blood and tissues. Therefore, whether or not macrophages could be induced in the *in vitro* culture by oxidized LDL (oxLDL) or advanced glycation end products (AGE), two abnormal metabolites associated with dyslipidemia and diabetes, was determined. As shown, macrophages of wild type mice significantly upregulated their expression of multiple NKG2D ligands at both transcript and protein levels when cultured in the presence of oxLDL and AGE (Fig. 3F-H). Referring to Fig. 3F, after a 2-day culture, mRNAs were isolated from the differently treated macrophages, reverse transcribed to generate cDNAs, which were five-fold serially diluted and subject to PCRs for detection of NKG2D ligands Rae-1δ, Rae-1ε and H60b. Referring to Fig. 3G, macrophages treated in the same way as in Fig. 3F were lysed, separated on PAGE gels and analyzed by Western blot with an anti-Rae1 antibody that reacts with all isoforms of Rae-1 molecules. Referring to Fig. 3H, wild type macrophages cultured with oxLDL or AGE also had higher cell surface levels of the Rae-1 staining based on flow cytometry analysis. The Rae-1 staining could be competed off by non-colored "cold" anti-RAE-1 antibodies, confirming a specific upregulation of the NKG2D ligand (Fig. 3H).

Taken together, these experiments demonstrated that multiple ligands for NKG2D were upregulated in arteries of mice with lipid and/or glucose metabolism disorders. The profound upregulation of the NKG2D ligands in the plaque lesions suggested a direct role of the NKG2D ligands in propagating vascular inflammation and atherosclerosis. Interestingly, only cells in the plaques, such as macrophages, had the most profound upregulation of transcripts and proteins of the NKG2D ligands. Considering that many cells in the plaques are activated in response to the lipid components, these data suggested that the NKG2D ligand upregulation is likely induced through both stress-responsible and immune activation pathways in the different types of cells.

### Example 3: Suppression of plague formation in ApoE-null mice by blockage of the NKG2D/ligand interaction with monoclonal anti-NKG2D antibodies or NKG2D knockout

The NKG2D ligand upregulation in arteries, especially on the plaques, might activate NKG2D-expressing immune cells to promote the vascular inflammation and atherosclerosis. To test this, monoclonal anti-NKG2D antibody (clone MI-6) was injected into diabetic ApoE-null mice to block the potential NKG2D/ligands interaction and whether such a blockage could suppress the plaque formation was determined. Injection of the anti-NKG2D antibody has been shown to block the NKG2D/ligand interaction in mice without deleting NKG2D-expressing immune cells (Jamieson and Raulet, unpublished). Six-week old male homozygous ApoE-null mice of C57BL/6 background were induced diabetic by STZ injection to accelerate the plaque formation. One week and three days before the STZ administration, 200 micrograms/injection of the monoclonal anti-NKG2D antibody (clone MI-6, rat IgG2a) were intraperitoneally injected into the ApoE-null mice. In the control group, the mice were treated in the same way except that they were given isotype-matched control antibody instead of anit-NKG2D antibody. The mice were kept on normal chow for eight weeks after induction of the diabetes, during which period anti-NKG2D or control antibodies were given to the mice once a week throughout the experimental duration. One week after the last antibody injection, the mice were sacrificed and analyzed for atherosclerotic lesions in aorta.

Compared to those injected with isotype-matched control antibodies, the diabetic ApoE-null mice injected with the anti-NKG2D antibody had a dramatic reduction in the plaque formation (Fig. 4). Of all the seven anti-NKG2D antibody treated and eight control antibody treated mice (in three separate experiments), sizes of the plaques in the NKG2D-antibody treated mice were always significantly smaller than those of the control antibody treated mice (Fig. 4A). To further determine the extent of the suppression of the plaque formation, en face staining of aortae was performed with Oil Red O dye for deposition of lipid contents in five of the seven anti-NKG2D antibody-treated mice and six of the eight control antibody-treated mice (of two separate experiments). There was enormous amounts of oil red O staining in the arch areas of aortae of the control antibody-treated mice (Fig. 4B, the three on the right). In contrast, the staining was much less intense in the anti-NKG2D antibody-treated mice (Fig. 4B, the two on the left), further demonstrating that the anti-NKG2D antibody blockage suppressed lipid deposition and plaque formation. On average, there was about a five-fold reduction in the Oil Red O staining positive area in the anti-NKG2D antibody treated mice compared to the controls (Fig. 5A).

Confirming that the NKG2D-antibody treatment specifically blocked NKG2D without induction of deletion of NKG2D-expressing cells, it was found that there were equal numbers of NK1.1+ NK cells and NKT cells in the anti-NKG2D and control antibody treated mice and the NK cells of the anti-NKG2D antibody treated mice express NKG2D normally. In addition, the anti-NKG2D antibody treatment did not result in global anergy of the NKG2D-expressing immune cells. In the *in vitro* assays, NK cells of the anti-NKG2D treated mice were responsive to the anti-NK1.1 antibody stimulation to express interferon-γ just as efficiently as those of control antibody treated mice. Together, these experiments demonstrated that the NKG2D/ligand interaction-mediated immune activation played an important role in the atherosclerosis and blockage of such an interaction suppressed the atherosclerosis.

To prove the critical role of NKG2D/ligand interaction in the atherosclerosis, ApoE-/- mice were crossed with NKG2D knockout mice to generate ApoE/NKG2D double knockout (ApoE-/-KLRK1^{-/-}) mice. Two models to test the effect of the ApoE/NKG2D double knockout on the development of atherosclerosis were tested. The first model is the STZ-induced diabetes, as used in the anti-NKG2D antibody blockage assay (Fig. 4). The second model is Western diet-accelerated atherosclerosis in which mice were fed with a high fat diet, starting at age six weeks. Eight to ten weeks after the onset of diabetes or start of the Western diet, the ApoE-/-KLRK1-/- mice were analyzed for the plaque formation on aorta by the en face Oil Red O staining. Compared to the ApoE-/-KLRK1+/+ mice treated in the same ways, both the diabetic and Western-diet fed ApoE-/-KLRK1-/- mice had dramatically reduced plaque sizes based on Oil Red O staining positive areas (Fig. 5B and C). These findings provided genetic proof of the important role of NKG2D/ligand interaction in atherosclerosis. In addition, they also provide evidence on wide involvement of this interaction in both diabetes and dyslipidemia-associated atherosclerosis, suggesting potential usage of targeting this molecular interaction for treatment and prevention of atherosclerosis of various origins.

### Example 4: Blockage of NKG2D/ligand interaction decreased expression of pro-inflammatory cytokines in atherosclerotic ApoE-null mice

Since the NKD2D/ligand interaction mediates immune activation in multiple NKG2D-expressing immune cell types, the suppression of the atherosclerosis by the anti-NKG2D antibody blockage suggested that it functioned through inhibiting inflammation. To test this, sera from the anti-NKG2D and control antibody treated diabetic ApoE-/- mice were collected at the time of plaque analysis and levels of multiple pro-inflammatory cytokines in the sera were analyzed, along with sera of other control mice, using a mouse pro-inflammatory-7 plex kit (Meso Scale Discovery). Of 7 cytokines assessed, five (IL-6, TNF-α, IFN-γ, IL-10 and IL-12p70) were reduced in the anti-NKG2D antibody treated mice, compared to the control antibody treated mice (Fig. 6). Most noticeably, IL-6 was dramatically reduced to the levels of aged-matched healthy wild-type B6 mice or young ApoE-/- mice that have not had dyslipidemia or diabetes (more than 10 fold reduction, P<0.001). TNF-α was reduced to non-detectable levels in the anti-NKG2D treated mice. These data indicated that the antibody blockage of NKG2D/ligand interaction suppressed the inflammation in the diabetic ApoE-/- mice to prevent the atherosclerosis.

The reduction of IL-6 in the anti-NKG2D antibody treated mice correlates with the reverse relationship of soluble MICA and IL-6 in the serum of diabetic patients (Fig. 1). Considering both the anti-NKG2D antibody and sMICA function by interfering with NKG2D mediated immune activation, high levels of sMICA in the human patients might be a naturally occurring mechanism for suppression of inflammation.

The decreased production of pro-inflammatory cytokines was also observed in the KLRK1-/-ApoE-/- mice. Compared to diabetic or western diet fed NKG2D-sufficient ApoE-/- mice, the similarly treated KLRK1-/-ApoE-/- mice had reduced serum levels of most pro-inflammatory cytokines (Fig. 7). Multiple inflammatory cytokines (IL-6, IFN-γ, IL-12 and TNF-α) were also reduced in the serum of NKG2D-antibody-treated KLRK1-/-ApoE-/- mice. Notably, IL-6 and IFN-γ were dramatically reduced in all the models. Together, these results demonstrate that preventing the NKG2D/ligand interaction suppressed inflammation and reduced atherosclerosis.

### Example 5: Preventing the NKG2D/ligand interaction alleviated abnormal metabolic conditions in ApoE-/- mice

Besides reducing the atherosclerosis and inflammation, preventing the NKG2D/ligand interaction also considerably alleviated metabolic disorders. In striking contrast to the turbid appearance of sera from western-diet fed ApoE-/- mice, those of similarly fed KLRK1-/-ApoE-/- mice were clear (Fig. 8A), suggesting reduced accumulation of lipid components in their blood. Confirming this, serum levels of cholesterol and triglycerides were significantly lower in KLRK1-/-ApoE-/- mice than in the ApoE-/- mice (Fig. 8B). Considering that the abnormal lipid metabolic condition contributes to atherosclerosis directly and promotes inflammation, these results suggested that the NKG2D/ligand interaction propagates the positive feedback cycle of inflammation and metabolic dysfunction to sustain atherosclerotic progression.

In summary, these results demonstrate that abnormal metabolic condition-associated upregulation of NKG2D ligands is critically involved in atherosclerosis and inhibiting the NKG2D/ligand interaction suppressed the disease progression by reducing the inflammation and alleviating abnormal metabolic disorders, suggesting it as an attractive therapeutic target in treatment against atherosclerosis.

### Example 6: Preventing the NKG2D/ligand interaction suppressed liver inflammation in ApoE-/- mice

Reduced serum levels of cholesterol and triglycerides in the Western diet fed NKG2D-knockout ApoE^{-/-} mice suggested that the NKG2D/ligand mediated immune activation aggravates the metabolic dysfunctions. Since the liver is the major organ of lipid metabolism, the NKG2D/ligand interaction mediated inflammation likely aggravated the abnormal metabolic conditions by impairing the function. Indeed, compared to ApoE^{-/-} mice, NKG2D-knockout ApoE^{-/-} mice had significantly lower activity of serum alanine aminotransferase (ALT) (Fig. 9), indicating an alleviated liver dysfunction. The ALT activities in the serum were determined using a kit from Bioo Scientific (Austin, TX) according to the manufacturer's instruction. For each group, at least 5 mice were used. **P<0.01.

It was tested whether preventing the NKG2D/ligand interaction reduced the amount of liver inflammation. Compared to those of ApoE^{-/-} mice, liver explants of NKG2D-knockout ApoE^{-/-} mice, cultured *ex vivo*, produced significantly less IL-6, indicating a reduced inflammation (Fig. 10A). In addition, compared to those of ApoE^{-/-} mice, numbers of various immune cells, including macrophages, NKT and NK cells, were all reduced in livers of NKG2D-knockout ApoE^{-/-} mice, (Fig. 10B), indicating that preventing the NKG2D/ligand interaction indeed reduced liver inflammation.

What immune cells were affected by the NKG2D/ligand interaction to contribute to the liver inflammation was investigated. Like the atherosclerotic aortae, livers of ApoE^{-/-} mice had high upregulation of NKG2D ligands (Fig. 11A). Both macrophages and hepatocytes of livers had upregulated Rae-1 expression in ApoE^{-/-}mice, compared to the wild-type controls (Fig. 11B-D). Corresponding to the upregulated ligands in the livers of Western diet fed ApoE^{-/-} mice, NKG2D-expressing immune cells, such as NK and particularly NK T cells, of the livers of these mice exhibited considerable cytokine production, including IFN-γ and IL-4, which was markedly attenuated when the mice also lacked NKG2D (Fig. 11E and F). In contrast, the liver macrophages, which do not express NKG2D, produced high levels of IL-6 in ApoE^{-/-} mice whether or not the mice expressed NKG2D (Fig. 11G). As already noted, however, the number of macrophages was reduced substantially in Klrk1^{-/-}ApoE^{-/-} mice, indicating that macrophages were indirectly influenced by NKG2D mediated inflammation. These findings demonstrate that abnormal metabolic conditions induce the upregulation of NKG2D ligands, particularly in tissues where abnormal metabolites accumulate, such as atherosclerotic plaques and the liver, and therefore the NKG2D/ligand interaction is a useful intervention target against atherosclerosis.

### Example 7: Preventing the NKG2D/ligand interaction reduced glucose levels in Western diet fed ApoE-/- mice

In one experiment, 8-week old ApoE-/- and NKG2D-deficienct ApoE-/- Klrk1-/- mice were fed on the Western diet for 3 months. Blood sera were then collected and assessed for the glucose levels at the end of the feeding regimen. The ApoE-/-Klrk-/- mice on average had significantly lower glucose levels than ApoE-/- mice after feeding on the Western diet for three months (Fig. 12), suggesting that preventing the NKG2D/ligand interaction could suppress the hyperglycemia progression.

### Example 8: Anti-NKG2D antibody prevents the development and progression of hyperglycaemia and diabetes in a rodent model of type 2 diabetes

To further determine the effect of blocking NKG2D on the development and progression of type 2 diabetes, an anti-NKG2D antibody was administered to *Psammomys obesus* (also known as sand rats), a gerbil animal model of type 2 diabetes.

Male and female *P. obesus* (Harlan, Jerusalem, Israel), were fed low energy (2.4 kcal/g) chow until the age of 9 weeks when they were transferred to an ad libitum high energy (3.1 kcal/g) diet during which body-weight (BW) and morning blood glucose (mBG) was followed for up to 10 days (induction period). The animals that showed increased mBG levels, defined as mBG >10 mmol/L on two consecutive readings, were transferred back to low energy diet and were used in the actual study (prevention) 10 days later when their mBG levels were back to non-diabetic levels. The animals that did not show any increase in mBG during the induction period were sacrificed and not used further.

*P. obesus* were treated with vehicle (N=19) or NKG2D-PE antibody clone CX5) (N=9), once weekly by intra-peritoneal injection. The NKG2D rodent antibody formulation includes CX5 OP001 - ca. at 10 EU/ml or 0.96 mg/ml. One vial of 45 ml, dissolved in PBS buffer was kept at 4°C and the dose volume used was 0.52 ml/kg or 0.5 mg/kg.

The study was conducted over 6 weeks during which mBG and BW were followed regularly. For the determination of mBG (mmol/I), blood samples were taken from the tail tip capillary into 10µl glass capillary tubes and immediately suspended in buffer (500µl of Biosen analysis buffer) in eppendorf vials and analyzed for glucose on the test day.

During the study, five animals in the vehicle group and one in the NKG2D antibody group had to be sacrificed due to severely elevated mBG and ketoacidosis. The study was approved by the Animal Experiments Inspectorate, Ministry of Justice, Denmark.

As shown in Fig. 13, six weeks after the first dose of vehicle or NKG2D antibody, all the animals in the vehicle had become severely diabetic with a mean mBG of 14.5 ± 2.6 mM (mean ± sem) while the animals treated with the NKG2D antibody had remained normoglycaemic (mBG<8 mM) and had a significantly lower mBG of 8.0 ± 1.9 mM, p<0.0001 (mean ± sem). There was no difference in BW gain in the two treatment groups. These findings demonstrate that NKG2D plays a very important role in the development of overt type 2 diabetes (probably through reduced inflammation, insulin resistance and beta cell failure) in a rodent animal model that has many of the same features as humans with type 2 diabetes. By blocking the effects of NKG2D with an antibody, the high-energy diet induced development of type 2 diabetes in *P.obesus* was completely abolished.

To confirm the specificity of the anti-NKG2D antibody, flow cytometric analysis of the NKG2D antibody binding to blood cells from *P. obesus* was performed.

Briefly, EDTA-stabilized blood from sand rats was stained in 100 µl aliquots with antibodies against NKG2D or isotype controls: anti-mouse NKG2D-PE (clone CX5), rat IgG1-PE isotype control, anti-human NKG2D-PE (clone 1D11), mouse IgG1-PE isotype control and anti-human NKG2D-PE (clone ON72). Blood was then lyzed, fixed and washed using BD FACS lysing/fixing solution and PBS and binding of PE-conjugated antibodies was measured on a LSRII flow cytometer. FSC/SSC was used to identify non-granulocytes.

As shown by the flow cytometric analysis in Fig. 14, a dose-dependent binding was observed for the anti-mouse NKG2D-PE antibody (clone CX5) to NK cells in sand rat blood. This binding was specific as there was no binding of the isotype control antibodies (rat IgG1-PE isotype control (R3-34) and mouse IgG1-PE isotype control (MOPC-21)) or the anti-human NKG2D antibodies (clone 1 D11 and clone ON72).

The results demonstrate the usefulness of targeting NKG2D for the treatment of type 2 diabetes and hyperglycemia.

### Materials and Methods

*Mouse models and human patients:* ApoE^{-/-} mice on the C56BL/6 background were purchased from Jackson Lab. Klrk1^{-/-} mice were previously described. Klrk1^{-/-}ApoE^{-/-} mice were generated by crossing ApoE^{-/-} and Klrk1^{-/-} mice and intercrossing the pups. To accelerate atherosclerosis, six-week old males were fed on a Western diet al libido or injected with streptozotocin (STZ) to induce diabetes (Park et al., Nat Med, 4:1025-1031 (1998)) and analyzed 8-10 weeks after initiation of the treatments. For the NKG2D antibody blockage experiment, ApoE^{-/-}mice were injected with the monoclonal NKG2D antibody (clone MI-6, rat IgG2a) (Jamieson et al., Immunity, 17:19-29 (2002)) one week and three days before the STZ administration and weekly afterwards for eight weeks (200 µg/1st injection and 100 µg/injection of the others).

*Antibodies and reagents:* The NKG2D antibody (clone MI-6) was prepared in house. Pan-Rae-1 antibody was purchased from R&D Systems while the others were from BD Biosciences or eBioscience or indicated in the following relevant sections.

*En face Oil Red* O *staining:* Aortae of differently treated mice were opened up, fixed in 10% formalin and stained with Oil Red O dye. Sizes of Oil Red O staining positive plaque areas were calculated based on digital pictures of the stained aortae using Adobe Photoshop.

*Immunohistochemical staining:* Cryosections of mouse aortic plaques were stained with polyclonal goat anti-mouse Rae-1 or H60 antibody (R&D Systems and Santa Cruz Biotechnology respectively), followed by horseradish peroxidase (HRP)-conjugated donkey anti-goat IgG antibody and ABC-HRP staining system (brown color) (Santa Cruz Biotechnology). The sections were further stained with monoclonal rat anti-mouse CD68 antibody (Serotec), followed by Alkaline Phosphotase (AP)-conjugated donkey anti-rat antibody and ABC-AP staining kit (blue color) (Vector Laboratory). Adjacent cryosections of human aortic plaques were stained with a monoclonal mouse anti-human MICA/B antibody (clone 6D4, eBioscience), anti-Mac-3 or anti-CD31 antibody.

*Cellular isolation:* Mononucleocytes, endothelial cells and hepatocytes were isolated from aortae or livers according to the reported procedures. The isolated cells were counted and used for various analyses.

*Flow cytometry:* Cells were stained with proper combination of fluorescently labeled antibodies and analyzed by flow cytometry using the flow cytometer FC500 (Beckman Counter).

*Semi-quantitative and real-time RT-PCR analysis:* RNA was analyzed for transcripts of Rae-1δ, Rae-1ε and H60b by semi-quantitative radioactive or real-time RT-PCR.

*In vitro treatment of macrophages:* Peritoneal macrophages were cultured *in vitro* for two days in media containing oxLDL (10 µg/ml), natural LDL (nLDL) (10 µg/ml), AGE (200 µg/ml) or LPS (200 ng/ml), and analyzed for the expression of NKG2D ligands.

*Western blot analysis for total Rae-1 proteins:* The macrophages were lysed, separated on PAGE gels, transferred to PEGF membrane, blotted with anti-Rae1 antibody (C20, Santa Cruz Biotechnology) and developed with a SEL system.

*Multiplex analysis of serum cytokines:* Mouse sera were analyzed directly on a mouse prolnflammatory-7plex kit (IL-6, TNF-α, IFN-γ, IL-12p70, IL-10, IL-1β and KC/CXCL1) (Meso Scale Discovery, Gaithersburg, MD).

*Assessment of cholesterol and triglyceride levels in serum:* Levels of cholesterol and triglycerides were analyzed on VetTest® Chemistry Analyzer (IDEXX Laboratories, Inc. Maine).

*ALT activity analysis:* The ALT activities in the serum were determined using a kit from Bioo Scientific (Austin, TX) according to the manufacturer's instruction.

*Ex vivo culture of liver explants:* PBS-perfused livers were excised from Western diet fed ApoE^{-/-} or Klrk1^{-/-}ApoE^{-/-} mice. Equal weights of excised livers were cut into about 1 mm³ cubes and cultured in media for 1 or 3 days. The culture media were recovered and analyzed for cytokines by ELISA.

*Intracellular cytokine staining:* Mononucleocytes isolated from livers were cultured in media overnight in presence of brofeldin A and analyzed by the intracellular cytokine staining for the production of IL-6, IFN-γ and IL-4 in gated subsets of immune cells according to the manufacturers' instructions (eBioscience and Biolegend).

*ELISA detection of soluble MICA:* Sera of type 2 diabetic and cancer patients were assayed for MICA proteins using an ELISA kit (R&D Systems, Minneapolis, MN).

*Statistical analyses:* All data are expressed as means ± s.e.m. Statistical significance was determined with a two-tail student T test. P < 0.05 is considered statistically significant.

All references, including publications, patent applications and patents, cited herein are hereby incorporated by reference to the same extent as if each reference was individually and specifically indicated to be incorporated by reference and was set forth in its entirety herein.

Any combination of the above-described elements in all possible variations thereof is encompassed by the invention unless otherwise indicated herein or otherwise clearly con-tradicted by context.

The terms "a" and "an" and "the" and similar referents as used in the context of de-scribing the invention are to be construed to cover both the singular and the plural, unless otherwise indicated herein or clearly contradicted by context.

The use of any and all examples, or exemplary language (e.g., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

The description herein of any aspect or embodiment of the invention using terms such as "comprising", "having", "including" or "containing" with reference to an element or elements is intended to provide support for a similar aspect or embodiment of the invention that "consists of", "consists essentially of", or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

This invention includes all modifications and equivalents of the subject matter recited in the aspects or claims presented herein to the maximum extent permitted by applicable law.

## Claims

1. An agent for use in a method of treating type 2 diabetes, wherein the agent blocks the NKG2D ligand binding interaction in a subject, and wherein the NKG2D ligand is selected from the group consisting of MICA/B, ULBP/RAET1, Multl, Rae-1δ Rae-1ε and H60b.

2. An agent for use in a method of (a) treating a condition that may be regulated or normalized via inhibition of NKG2D, wherein the condition is selected from the group consisting of cardiovascular disease, high cholesterol level, high triglyceride level, dyslipidemia, hyperglycemia, diabetic retinopathy, metabolic syndrome, kidney failure and poor blood circulation, or (b) suppressing atherosclerotic plaque formation, and wherein the agent blocks the interaction of NKG2D and NKG2D ligand in a subject, and wherein the NKG2D ligand is selected from the group consisting of MICA/B, ULBP/RAET1, Multl, Rae-1δ, Rae-1ε and H60b.

3. The agent for use according to claim 1 or 2, wherein the subject is human.

4. The agent for use according to any of one of claims 1-3, wherein the agent is selected from the group consisting of soluble NKG2D, an antibody or antigen-binding fragment thereof that specifically binds NKG2D, an NKG2D ligand and an inhibitor of NKG2D ligand activity or expression.

5. The agent for use according to claim 4, wherein the agent is an antibody or antigen-binding fragment thereof that specifically binds NKG2D.

6. The agent for use according to claim 5, wherein the antibody or antigen-binding fragment thereof is human or humanized.

7. The agent for use according to claim 5, wherein the antibody or antigen-binding fragment thereof binds human NKG2D (hNKG2D).

8. The agent for use according to claim 5, wherein the antibody or antigen-binding fragment thereof reduces NKG2D-mediated activation or signalling of an NKG2D-expressing cell.

9. The agent for use according to claim 5, wherein the antibody or antigen-binding fragment thereof competes with at least one NKG2D ligand in binding to NKG2D.

10. The agent for use according to claim 9, wherein the NKG2D ligand is MICA/B.

11. The agent for use according to any of one of claims 1-10, wherein administration of the agent results in at least one of the following in the subject: reduces blood glucose levels, improves glucose tolerance, lowers insulin resistance, reduces body weight, lowers blood pressure, lowers inflammation or reduces metabolic dysfunctions.

12. The agent for use according to any of one of claims 1-11, wherein the agent is to be administered intravenously, intraperitoneal or subcutaneously.

13. The agent for use according to any one of claims 1-11, further comprising at least one additional agent selected from the group consisting of antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity.

14. Use of an agent in the manufacture of a medicament for treating type 2 diabetes, wherein the agent blocks the NKG2D ligand binding interaction in a subject, and wherein the NKG2D ligand is selected from the group consisting of MICA/B, ULBP/RAET1, Multl, Rae-1δ, Rae-1ε and H60b.

15. Use of an agent in the manufacture of a medicament for (a) treating a condition that may be regulated or normalized via inhibition of NKG2D, wherein the condition is selected from the group consisting of, cardiovascular disease, high cholesterol level, high triglyceride level, dyslipidemia, hyperglycemia, diabetic retinopathy, metabolic syndrome, kidney failure and poor blood circulation, or (b) suppressing atherosclerotic plaque formation, and wherein the agent blocks the interaction of NKG2D and NKG2D ligand in a subject, and wherein the NKG2D ligand is selected from the group consisting of MICA/B, ULBP/RAET1, Multl, Rae-1δ, Rae-1ε and H60b.

## Patentansprüche

1. Mittel zur Verwendung in einem Verfahren zur Behandlung von Typ-2-Diabetes, wobei das Mittel die NKG2D-Ligandenbindungsinteraktion in einem Individuum blockiert und wobei der NKG2D-Ligand ausgewählt ist aus der Gruppe, bestehend aus MICA/B, ULBP/RAET1, Multl, Rae-1δ, Rae-1ε und H60b.

2. Mittel zur Verwendung in einem Verfahren zur (a) Behandlung eines Zustands, der über die Hemmung von NKG2D reguliert oder normalisiert werden kann, wobei der Zustand ausgewählt ist aus der Gruppe, bestehend aus einer Herz-Kreislauferkrankung, einem hohen Cholesterinspiegel, einem hohen Triglyceridspiegel, Dyslipidämie, Hyperglykämie, diabetischer Retinopathie, metabolischem Syndrom, Nierenversagen und einem schlechten Blutkreislauf oder (b) Unterdrückung einer arteriosklerotischen Plaquebildung und wobei das Mittel die Interaktion von NKG2D und NKG2D-Ligand in einem Individuum blockiert und wobei der NKG2D-Ligand ausgewählt aus der Gruppe bestehend aus MICA/B, ULBP/RAET1, Multl, Rae-1δ, Rae-1ε und H60b.

3. Mittel zur Verwendung nach Anspruch 1 oder 2, wobei das Individuum menschlich ist.

4. Mittel zur Verwendung nach einem der Ansprüche 1-3, wobei das Mittel ausgewählt ist aus der Gruppe, bestehend aus löslichem NKG2D, einem Antikörper oder einem Antigen-bindenden Fragment davon, das spezifisch NKG2D bindet, einem NKG2D-Liganden und einem Hemmer der NKG2D-Ligandenaktivität oder -expression.

5. Mittel zur Verwendung nach Anspruch 4, wobei das Mittel ein Antikörper oder ein Antigen-bindendes Fragment davon ist, der bzw. das spezifisch NKG2D bindet.

6. Mittel zur Verwendung nach Anspruch 5, wobei der Antikörper oder das Antigen-bindende Fragment davon menschlich oder humanisiert ist.

7. Mittel zur Verwendung nach Anspruch 5, wobei der Antikörper oder das Antigen-bindende Fragment davon menschliches NKG2D (hNKG2D) bindet.

8. Mittel zur Verwendung nach Anspruch 5, wobei der Antikörper oder das Antigen-bindende Fragment davon die durch NKG2D vermittelte Aktivierung oder Signalisierung einer NKG2D exprimierenden Zelle verringert.

9. Mittel zur Verwendung nach Anspruch 5, wobei der Antikörper oder das Antigen-bindende Fragment davon mit mindestens einem NKG2D-Liganden bei der Bindung an NKG2D konkurriert.

10. Mittel zur Verwendung nach Anspruch 9, wobei der NKG2D-Ligand MICA/B ist.

11. Mittel zur Verwendung nach einem der Ansprüche 1-10, wobei die Verabreichung des Mittels bei dem Individuum mindestens eines der Folgenden zur Folge hat: verringert den Blutzuckerspiegel, verbessert die Glucosetoleranz, senkt die Insulinresistenz, verringert das Körpergewicht, senkt den Blutdruck, verringert Entzündungen oder verringert Stoffwechselstörungen.

12. Mittel zur Verwendung nach einem der Ansprüche 1-11, wobei das Mittel intravenös, intraperitoneal oder subkutan verabreicht werden soll.

13. Mittel zur Verwendung nach einem der Ansprüche 1-11, ferner umfassend mindestens ein zusätzliches Mittel, ausgewählt aus der Gruppe, bestehend aus Antidiabetika, Mitteln gegen Fettleibigkeit, appetitregulierenden Mitteln, Antihypertensiva, Mitteln für die Behandlung und/oder Vorbeugung von Komplikationen, die durch Diabetes entstehen oder damit assoziiert sind und Mitteln für die Behandlung und/oder Vorbeugung von Komplikationen und Störungen, die durch Fettleibigkeit entstehen oder damit assoziiert sind.

14. Verwendung eines Mittels bei der Herstellung eines Medikaments zur Behandlung von Typ-2-Diabetes, wobei das Mittel die NKG2D-Ligandenbindungsinteraktion in einem Individuum blockiert und wobei der NKG2D-Ligand ausgewählt ist aus der Gruppe, bestehend aus MICA/B, ULBP/RAET1, Multl, Rae-1δ, Rae-1ε und H60b.

15. Verwendung eines Mittels bei der Herstellung eines Medikaments zur (a) Behandlung eines Zustands, der über die Hemmung von NKG2D reguliert oder normalisiert werden kann, wobei der Zustand ausgewählt ist aus der Gruppe, bestehend aus einer Herz-Kreislauferkrankung, einem hohen Cholesterinspiegel, einem hohen Triglyceridspiegel, Dyslipidämie, Hyperglykämie, diabetischer Retinopathie, metabolischem Syndrom, Nierenversagen und einem schlechten Blutkreislauf oder (b) Unterdrückung einer arteriosklerotischen Plaquebildung und wobei das Mittel die Interaktion von NKG2D und NKG2D-Ligand in einem Individuum blockiert und wobei der NKG2D-Ligand ausgewählt ist der Gruppe, bestehend aus MICA/B, ULBP/RAET1, Multl, Rae-1δ, Rae-1ε und H60b.

## Revendications

1. Agent pour une utilisation dans un procédé de traitement du diabète de type 2, dans lequel l'agent bloque l'interaction de liaison du ligand de NKG2D chez un sujet, et dans lequel le ligand de NKG2D est choisi dans le groupe composé de MICA/B, ULBP/RAET1, Multl, Rae-1δ, Rae-1ε et H60b.

2. Agent pour une utilisation dans un procédé de (a) traitement d'une pathologie qui peut être régulée ou normalisée par l'inhibition de NKG2D, dans lequel la pathologie est choisie dans le groupe composé de maladies cardiovasculaires, hypercholestérolémie, hypertriglycéridémie, dyslipidémie, hyperglycémie, rétinopathie diabétique, syndrome métabolique, insuffisance rénale et mauvaise circulation sanguine, ou (b) suppression de la formation des plaques d'athérome, et dans lequel l'agent bloque l'interaction de NKG2D et du ligand de NKG2D chez un sujet, et dans lequel le ligand de NKG2D est choisi dans le groupe composé de MICA/B, ULBP/RAET1, Multl, Rae-1δ, Rae-1ε et H60b.

3. Agent pour une utilisation selon la revendication 1 ou 2, dans lequel le sujet est humain.

4. Agent pour une utilisation selon l'une quelconque des revendications 1 à 3, dans lequel l'agent est choisi dans le groupe composé de NKG2D soluble, un anticorps ou un fragment de liaison de l'antigène de celui-ci qui lie spécifiquement NKG2D, un ligand de NKG2D et un inhibiteur de l'activité ou de l'expression du ligand de NKG2D.

5. Agent pour une utilisation selon la revendication 4, dans lequel l'agent est un anticorps ou un fragment de liaison de l'antigène de celui-ci qui lie spécifiquement NKG2D.

6. Agent pour une utilisation selon la revendication 5, dans lequel l'anticorps ou le fragment de liaison de l'antigène de celui-ci est humain ou humanisé.

7. Agent pour une utilisation selon la revendication 5, dans lequel l'anticorps ou le fragment de liaison de l'antigène de celui-ci lie le NKG2D humain (hNKG2D).

8. Agent pour une utilisation selon la revendication 5, dans lequel l'anticorps ou le fragment de liaison de l'antigène de celui-ci réduit l'activation ou la signalisation médiée par NKG2D d'une cellule exprimant NKG2D.

9. Agent pour une utilisation selon la revendication 5, dans lequel l'anticorps ou le fragment de liaison de l'antigène de celui-ci entre en compétition avec au moins un ligand de NKG2D dans la liaison à NKG2D.

10. Agent pour une utilisation selon la revendication 9, dans lequel le ligand de NKG2D est MICA/B.

11. Agent pour une utilisation selon l'une quelconque des revendications 1 à 10, dans lequel l'administration de l'agent produit au moins l'un des effets suivants chez le sujet : réduit la glycémie, améliore la tolérance au glucose, abaisse la résistance à l'insuline, réduit le poids corporel, abaisse la tension sanguine, abaisse l'inflammation ou réduit les dysfonctionnements métaboliques.

12. Agent pour une utilisation selon l'une quelconque des revendications 1 à 11, dans lequel l'agent doit être administré par voie intraveineuse, intrapéritonéale ou sous-cutanée.

13. Agent pour une utilisation selon l'une quelconque des revendications 1 à 11, comprenant en outre au moins un agent supplémentaire choisi dans le groupe composé d'agents antidiabétiques, d'agents anti-obésité, d'agents régulant l'appétit, d'agents antihypertenseurs, d'agents pour le traitement et/ou la prévention des complications résultant de ou associées au diabète et d'agents pour le traitement et/ou la prévention des complications et des troubles résultant de ou associés à l'obésité.

14. Utilisation d'un agent dans la fabrication d'un médicament pour le traitement du diabète de type 2, dans lequel l'agent bloque l'interaction de liaison du ligand de NKG2D chez un sujet, et dans lequel le ligand de NKG2D est choisi dans le groupe composé de MICA/B, ULBP/RAET1, Multl, Rae-1δ, Rae-1ε et H60b.

15. Utilisation d'un agent dans la fabrication d'un médicament pour (a) le traitement d'une pathologie qui peut être régulée ou normalisée par l'intermédiaire de l'inhibition de NKG2D, dans lequel la pathologie est choisie dans le groupe composé de maladies cardiovasculaires, hypercholestérolémie, hypertriglycéridémie, dyslipidémie, hyperglycémie, rétinopathie diabétique, syndrome métabolique, insuffisance rénale et mauvaise circulation sanguine, ou (b) la suppression de la formation des plaques d'athérome, et dans lequel l'agent bloque l'interaction de NKG2D et du ligand de NKG2D chez un sujet, et dans lequel le ligand de NKG2D est choisi dans le groupe composé de MICA/B, ULBP/RAET1, Multl, Rae-1δ, Rae-1ε et H60b.


### SEQUENCE LISTING

<110> The Penn State Research Foundation and The Regents of the University of California
<120> COMPOSITIONS, METHODS AND KITS FOR DETECTING AND TREATING ABNORMAL METABOLIC AND CARDIOVASCULAR DISEASES
<130> P025-0001PCT
<141> 2010-08-16
<150> US 61/234,425
   <151> 2009-08-17
<150> 61/304,113
   <151> 2010-02-12
<160> 40
<170> PatentIn version 3.5
<210> 1
   <211> 448
   <212> PRT
   <213> Homo sapiens
<400> 1
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Ser Ile Thr Ser Ser Ser Ser Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Arg Arg Tyr Phe Asp Trp Phe Pro Leu Asp Tyr Arg Gly
               100                 105                 110
   Gln Gly Thr Leu Val Thr Val Ser Ser Ala Ser Thr Lys Gly Pro Ser
           115                 120                 125
   Val Phe Pro Leu Ala Pro Cys Ser Arg Ser Thr Ser Glu Ser Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Lys Thr Tyr Thr Cys Asn Val Asp His
           195                 200                 205
   Lys Pro Ser Asn Thr Lys Val Asp Lys Arg Val Glu Ser Lys Tyr Gly
       210                 215                 220
   Pro Pro Cys Pro Pro Cys Pro Ala Pro Glu Phe Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser Gln Glu Asp Pro
               260                 265                 270
   Glu Val Gln Phe Asn Trp Tyr Val Asp Gly Val Glu Val His Asn Ala
           275                 280                 285
   Lys Thr Lys Pro Arg Glu Glu Gln Phe Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Gly Leu Pro Ser Ser Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Gln Glu Glu Met Thr Lys Asn Gln Val Ser Leu Thr Cys
           355                 360                 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Arg Leu Thr Val Asp Lys Ser
   405 410 415
   Arg Trp Gln Glu Gly Asn Val Phe Ser Cys Ser Val Met His Glu Ala
   420 425 430
   Leu His Asn His Tyr Thr Gln Lys Ser Leu Ser Leu Ser Leu Gly Lys
   435 440 445
<210> 2
   <211> 214
   <212> PRT
   <213> Homo sapiens
<400> 2
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Trp
               20                  25                  30
   Leu Ala Trp Tyr Gln Gln Lys Pro Glu Lys Ala Pro Lys Ser Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Gly Tyr Pro Tyr
                   85                  90                  95
   Thr Phe Gly Gln Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 3
   <211> 449
   <212> PRT
   <213> Homo sapiens
<400> 3
   Glu Val Gln Leu Val Glu Ser Gly Gly Gly Val Val Arg Pro Gly Gly
   1               5                   10                  15
   Ser Leu Arg Leu Ser Cys Ala Ala Ser Gly Phe Thr Phe Asp Asp Tyr
               20                  25                  30
   Gly Met Thr Trp Val Arg Gln Ala Pro Gly Lys Gly Leu Glu Trp Val
           35                  40                  45
   Ser Gly Ile Asn Trp Asn Gly Gly Ser Thr Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Arg Glu Arg Glu Leu Tyr Tyr Tyr Tyr Tyr Gly Leu Asp Val Trp
               100                 105                 110
   Gly Gln Gly Thr Thr Val Thr Val Ser Ser Ala Ser Thr Lys Gly Pro
           115                 120                 125
   Ser Val Phe Pro Leu Ala Pro Cys Ser Arg Ser Thr Ser Glu Ser Thr
       130                 135                 140
   Ala Ala Leu Gly Cys Leu Val Lys Asp Tyr Phe Pro Glu Pro Val Thr
   145                 150                 155                 160
   Val Ser Trp Asn Ser Gly Ala Leu Thr Ser Gly Val His Thr Phe Pro
                   165                 170                 175
   Ala Val Leu Gln Ser Ser Gly Leu Tyr Ser Leu Ser Ser Val Val Thr
               180                 185                 190
   Val Pro Ser Ser Ser Leu Gly Thr Lys Thr Tyr Thr Cys Asn Val Asp
           195                 200                 205
   His Lys Pro Ser Asn Thr Lys Val Asp Lys Arg Val Glu Ser Lys Tyr
       210                 215                 220
   Gly Pro Pro Cys Pro Pro Cys Pro Ala Pro Glu Phe Leu Gly Gly Pro
   225                 230                 235                 240
   Ser Val Phe Leu Phe Pro Pro Lys Pro Lys Asp Thr Leu Met Ile Ser
                   245                 250                 255
   Arg Thr Pro Glu Val Thr Cys Val Val Val Asp Val Ser Gln Glu Asp
               260                 265                 270
   Pro Glu Val Gln Phe Asn Trp Tyr Val Asp Gly Val Glu Val His Asn
           275                 280                 285
   Ala Lys Thr Lys Pro Arg Glu Glu Gln Phe Asn Ser Thr Tyr Arg Val
       290                 295                 300
   Val Ser Val Leu Thr Val Leu His Gln Asp Trp Leu Asn Gly Lys Glu
   305                 310                 315                 320
   Tyr Lys Cys Lys Val Ser Asn Lys Gly Leu Pro Ser Ser Ile Glu Lys
                   325                 330                 335
   Thr Ile Ser Lys Ala Lys Gly Gln Pro Arg Glu Pro Gln Val Tyr Thr
               340                 345                 350
   Leu Pro Pro Ser Gln Glu Glu Met Thr Lys Asn Gln Val Ser Leu Thr
           355                 360                 365
   Cys Leu Val Lys Gly Phe Tyr Pro Ser Asp Ile Ala Val Glu Trp Glu
       370                 375                 380
   Ser Asn Gly Gln Pro Glu Asn Asn Tyr Lys Thr Thr Pro Pro Val Leu
   385                 390                 395                 400
   Asp Ser Asp Gly Ser Phe Phe Leu Tyr Ser Arg Leu Thr Val Asp Lys
                   405                 410                 415
   Ser Arg Trp Gln Glu Gly Asn Val Phe Ser Cys Ser Val Met His Glu
               420                 425                 430
   Ala Leu His Asn His Tyr Thr Gln Lys Ser Leu Ser Leu Ser Leu Gly
           435                 440                 445
   Lys
<210> 4
   <211> 215
   <212> PRT
   <213> Homo sapiens
<400> 4
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Phe Thr Phe Gly Pro Gly Thr Lys Val Asp Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 5
   <211> 121
   <212> PRT
   <213> Homo sapiens
<400> 5
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Ser Ile Thr Ser Ser Ser Ser Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Arg Arg Tyr Phe Asp Trp Phe Pro Leu Asp Tyr Arg Gly
               100                 105                 110
   Gln Gly Thr Leu Val Thr Val Ser Ser
           115                 120
<210> 6
   <211> 108
   <212> PRT
   <213> Homo sapiens
<400> 6
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Trp
               20                  25                  30
   Leu Ala Trp Tyr Gln Gln Lys Pro Glu Lys Ala Pro Lys Ser Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Gly Tyr Pro Tyr
                   85                  90                  95
   Thr Phe Gly Gln Gly Thr Lys Leu Glu Ile Lys Arg
               100                 105
<210> 7
   <211> 122
   <212> PRT
   <213> Homo sapiens
<400> 7
   Glu Val Gln Leu Val Glu Ser Gly Gly Gly Val Val Arg Pro Gly Gly
   1               5                   10                  15
   Ser Leu Arg Leu Ser Cys Ala Ala Ser Gly Phe Thr Phe Asp Asp Tyr
               20                  25                  30
   Gly Met Thr Trp Val Arg Gln Ala Pro Gly Lys Gly Leu Glu Trp Val
           35                  40                  45
   Ser Gly Ile Asn Trp Asn Gly Gly Ser Thr Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Arg Glu Arg Glu Leu Tyr Tyr Tyr Tyr Tyr Gly Leu Asp Val Trp
               100                 105                 110
   Gly Gln Gly Thr Thr Val Thr Val Ser Ser
                       115                 120
<210> 8
   <211> 109
   <212> PRT
   <213> Homo sapiens
<400> 8
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Ser Pro
   85 90 95
   Phe Thr Phe Gly Pro Gly Thr Lys Val Asp Ile Lys Arg
   100 105
<210> 9
   <211> 5
   <212> PRT
   <213> Homo sapiens
<400> 9
   Ser Tyr Ser Met Asn
   1 5
<210> 10
   <211> 17
   <212> PRT
   <213> Homo sapiens
<400> 10
   Ser Ile Thr Ser Ser Ser Ser Tyr Ile Tyr Tyr Ala Asp Ser Val Lys
   1 5 10 15
   Gly
<210> 11
   <211> 12
   <212> PRT
   <213> Homo sapiens
<400> 11
   Asp Arg Arg Tyr Phe Asp Trp Phe Pro Leu Asp Tyr
   1 5 10
<210> 12
   <211> 11
   <212> PRT
   <213> Homo sapiens
<400> 12
   Arg Ala Ser Gln Gly Ile Ser Ser Trp Leu Ala
   1               5                   10
<210> 13
   <211> 7
   <212> PRT
   <213> Homo sapiens
<400> 13
   Ala Ala Ser Ser Leu Gln Ser
   1               5
<210> 14
   <211> 9
   <212> PRT
   <213> Homo sapiens
<400> 14
   Gln Gln Tyr Asn Gly Tyr Pro Tyr Thr
   1               5
<210> 15
   <211> 5
   <212> PRT
   <213> Homo sapiens
<400> 15
   Asp Tyr Gly Met Thr
   1               5
<210> 16
   <211> 17
   <212> PRT
   <213> Homo sapiens
<400> 16
   Gly Ile Asn Trp Asn Gly Gly Ser Thr Gly Tyr Ala Asp Ser Val Lys
   1               5                   10                  15
   Gly
<210> 17
   <211> 13
   <212> PRT
   <213> Homo sapiens
<400> 17
   Glu Arg Glu Leu Tyr Tyr Tyr Tyr Tyr Gly Leu Asp Val
   1               5                   10
<210> 18
   <211> 12
   <212> PRT
   <213> Homo sapiens
<400> 18
   Arg Ala Ser Gln Ser Val Ser Ser Ser Tyr Leu Ala
   1               5                   10
<210> 19
   <211> 7
   <212> PRT
   <213> Homo sapiens
<400> 19
   Gly Ala Ser Ser Arg Ala Thr
   1               5
<210> 20
   <211> 9
   <212> PRT
   <213> Homo sapiens
<400> 20
   Gln Gln Tyr Gly Ser Ser Pro Phe Thr
   1               5
<210> 21
   <211> 442
   <212> PRT
   <213> Homo sapiens
<400> 21
   Gln Val His Leu Gln Glu Ser Gly Pro Gly Leu Val Lys Pro Ser Glu
   1               5                   10                  15
   Thr Leu Ser Leu Thr Cys Thr Val Ser Asp Asp Ser Ile Ser Ser Tyr
   20 25 30
   Tyr Trp Ser Trp Ile Arg Gln Pro Pro Gly Lys Gly Leu Glu Trp Ile
   35 40 45
   Gly His Ile Ser Tyr Ser Gly Ser Ala Asn Tyr Asn Pro Ser Leu Lys
   50 55 60
   Ser Arg Val Thr Ile Ser Val Asp Thr Ser Lys Asn Gln Phe Ser Leu
   65 70 75 80
   Lys Leu Ser Ser Val Thr Ala Ala Asp Thr Ala Val Tyr Tyr Cys Ala
   85 90 95
   Asn Trp Asp Asp Ala Phe Asn Ile Trp Gly Gln Gly Thr Met Val Thr
   100 105 110
   Val Ser Ser Ala Ser Thr Lys Gly Pro Ser Val Phe Pro Leu Ala Pro
   115 120 125
   Cys Ser Arg Ser Thr Ser Glu Ser Thr Ala Ala Leu Gly Cys Leu Val
   130 135 140
   Lys Asp Tyr Phe Pro Glu Pro Val Thr Val Ser Trp Asn Ser Gly Ala
   145 150 155 160
   Leu Thr Ser Gly Val His Thr Phe Pro Ala Val Leu Gln Ser Ser Gly
   165 170 175
   Leu Tyr Ser Leu Ser Ser Val Val Thr Val Pro Ser Ser Ser Leu Gly
   180 185 190
   Thr Lys Thr Tyr Thr Cys Asn Val Asp His Lys Pro Ser Asn Thr Lys
   195 200 205
   Val Asp Lys Arg Val Glu Ser Lys Tyr Gly Pro Pro Cys Pro Pro Cys
   210 215 220
   Pro Ala Pro Glu Phe Leu Gly Gly Pro Ser Val Phe Leu Phe Pro Pro
   225 230 235 240
   Lys Pro Lys Asp Thr Leu Met Ile Ser Arg Thr Pro Glu Val Thr Cys
   245 250 255
   Val Val Val Asp Val Ser Gln Glu Asp Pro Glu Val Gln Phe Asn Trp
               260                 265                 270
   Tyr Val Asp Gly Val Glu Val His Asn Ala Lys Thr Lys Pro Arg Glu
           275                 280                 285
   Glu Gln Phe Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Gly Leu Pro Ser Ser Ile Glu Lys Thr Ile Ser Lys Ala Lys Gly
                   325                 330                 335
   Gln Pro Arg Glu Pro Gln Val Tyr Thr Leu Pro Pro Ser Gln Glu Glu
               340                 345                 350
   Met Thr Lys Asn Gln Val Ser Leu Thr Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Tyr Ser Arg Leu Thr Val Asp Lys Ser Arg Trp Gln Glu Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Leu Gly Lys
           435                 440
<210> 22
   <211> 215
   <212> PRT
   <213> Homo sapiens
<400> 22
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Trp Thr Phe Gly Gln Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 23
   <211> 450
   <212> PRT
   <213> Homo sapiens
<400> 23
   Glu Val Gln Leu Val Gln Ser Gly Ala Glu Val Lys Glu Pro Gly Glu
   1               5                   10                  15
   Ser Leu Lys Ile Ser Cys Lys Asn Ser Gly Tyr Ser Phe Thr Asn Tyr
               20                  25                  30
   Trp Val Gly Trp Val Arg Gln Met Pro Gly Lys Gly Leu Glu Trp Met
           35                  40                  45
   Gly Ile Ile Tyr Pro Gly Asp Ser Asp Thr Arg Tyr Ser Pro Ser Phe
       50                  55                  60
   Gln Gly Gln Val Thr Ile Ser Ala Asp Lys Ser Ile Asn Thr Ala Tyr
   65                  70                  75                  80
   Leu Gln Trp Ser Ser Leu Lys Ala Ser Asp Thr Ala Met Tyr Tyr Cys
                   85                  90                  95
   Gly Arg Leu Thr Met Phe Arg Gly Ile Ile Ile Gly Tyr Phe Asp Tyr
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Cys Ser Arg Ser Thr Ser Glu Ser
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Lys Thr Tyr Thr Cys Asn Val
           195                 200                 205
   Asp His Lys Pro Ser Asn Thr Lys Val Asp Lys Arg Val Glu Ser Lys
       210                 215                 220
   Tyr Gly Pro Pro Cys Pro Pro Cys Pro Ala Pro Glu Phe Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser Gln Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Phe Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Gly Leu Pro Ser Ser Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Gln Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Arg Leu Thr Val Asp
                   405                 410                 415
   Lys Ser Arg Trp Gln Glu Gly Asn Val Phe Ser Cys Ser Val Met His
               420                 425                 430
   Glu Ala Leu His Asn His Tyr Thr Gln Lys Ser Leu Ser Leu Ser Leu
           435                 440                 445
   Gly Lys
       450
<210> 24
   <211> 214
   <212> PRT
   <213> Homo sapiens
<400> 24
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 25
   <211> 115
   <212> PRT
   <213> Homo sapiens
<400> 25
   Gln Val His Leu Gln Glu Ser Gly Pro Gly Leu Val Lys Pro Ser Glu
   1               5                   10                  15
   Thr Leu Ser Leu Thr Cys Thr Val Ser Asp Asp Ser Ile Ser Ser Tyr
               20                  25                  30
   Tyr Trp Ser Trp Ile Arg Gln Pro Pro Gly Lys Gly Leu Glu Trp Ile
           35                  40                  45
   Gly His Ile Ser Tyr Ser Gly Ser Ala Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Asn Trp Asp Asp Ala Phe Asn Ile Trp Gly Gln Gly Thr Met Val Thr
               100                 105                 110
   Val Ser Ser
           115
<210> 26
   <211> 108
   <212> PRT
   <213> Homo sapiens
<400> 26
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Trp Thr Phe Gly Gln Gly Thr Lys Val Glu Ile Lys
               100                 105
<210> 27
   <211> 123
   <212> PRT
   <213> Homo sapiens
<400> 27
   Glu Val Gln Leu Val Gln Ser Gly Ala Glu Val Lys Glu Pro Gly Glu
   1               5                   10                  15
   Ser Leu Lys Ile Ser Cys Lys Asn Ser Gly Tyr Ser Phe Thr Asn Tyr
               20                  25                  30
   Trp Val Gly Trp Val Arg Gln Met Pro Gly Lys Gly Leu Glu Trp Met
           35                  40                  45
   Gly Ile Ile Tyr Pro Gly Asp Ser Asp Thr Arg Tyr Ser Pro Ser Phe
       50                  55                  60
   Gln Gly Gln Val Thr Ile Ser Ala Asp Lys Ser Ile Asn Thr Ala Tyr
   65                  70                  75                  80
   Leu Gln Trp Ser Ser Leu Lys Ala Ser Asp Thr Ala Met Tyr Tyr Cys
                   85                  90                  95
   Gly Arg Leu Thr Met Phe Arg Gly Ile Ile Ile Gly Tyr Phe Asp Tyr
               100                 105                 110
   Trp Gly Gln Gly Thr Leu Val Thr Val Ser Ser
           115                 120
<210> 28
   <211> 107
   <212> PRT
   <213> Homo sapiens
<400> 28
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Trp
                   85                  90                  95
   Thr Phe Gly Gln Gly Thr Lys Val Glu Ile Lys
               100                 105
<210> 29
   <211> 5
   <212> PRT
   <213> Homo sapiens
<400> 29
   Ser Tyr Tyr Trp Ser
   1               5
<210> 30
   <211> 16
   <212> PRT
   <213> Homo sapiens
<400> 30
   His Ile Ser Tyr Ser Gly Ser Ala Asn Tyr Asn Pro Ser Leu Lys Ser
   1               5                   10                  15
<210> 31
   <211> 7
   <212> PRT
   <213> Homo sapiens
<400> 31
   Trp Asp Asp Ala Phe Asn Ile
   1               5
<210> 32
   <211> 12
   <212> PRT
   <213> Homo sapiens
<400> 32
   Arg Ala Ser Gln Ser Val Ser Ser Ser Tyr Leu Ala
   1 5 10
<210> 33
   <211> 7
   <212> PRT
   <213> Homo sapiens
<400> 33
   Gly Ala Ser Ser Arg Ala Thr
   1 5
<210> 34
   <211> 9
   <212> PRT
   <213> Homo sapiens
<400> 34
   Gln Gln Tyr Gly Ser Ser Pro Trp Thr
   1 5
<210> 35
   <211> 5
   <212> PRT
   <213> Homo sapiens
<400> 35
   Asn Tyr Trp Val Gly
   1 5
<210> 36
   <211> 17
   <212> PRT
   <213> Homo sapiens
<400> 36
   Ile Ile Tyr Pro Gly Asp Ser Asp Thr Arg Tyr Ser Pro Ser Phe Gln
   1 5 10 15
   Gly
<210> 37
   <211> 14
   <212> PRT
   <213> Homo sapiens
<400> 37
   Leu Thr Met Phe Arg Gly Ile Ile Ile Gly Tyr Phe Asp Tyr
   1 5 10
<210> 38
   <211> 11
   <212> PRT
   <213> Homo sapiens
<400> 38
   Arg Ala Ser Gln Ser Val Ser Ser Tyr Leu Ala
   1 5 10
<210> 39
   <211> 7
   <212> PRT
   <213> Homo sapiens
<400> 39
   Asp Ala Ser Asn Arg Ala Thr
   1 5
<210> 40
   <211> 9
   <212> PRT
   <213> Homo sapiens
<400> 40
   Gln Gln Arg Ser Asn Trp Pro Trp Thr
   1 5